# EUROPEAN PATENT APPLICATION

(11) **EP 4 268 810 A1**
(43) Date of publication of application: **01.11.2023**
(21) Application number: 22170481.0
(22) Date of filing: 28.04.2022
(51) Int. Cl.: A61K 31/155, A61P 31/14

(54) **BROAD-SPECTRUM ANTIVIRAL AGENT COMPRISING PHENFORMIN**

(71) Applicant: Eberhard Karls Universität Tübingen, Medizinische Fakultät, 72074 Tübingen (DE); Christian-Albrechts-Universität zu Kiel, 24118 Kiel (DE)
(72) Inventor: Schindler, Michael, 72770 Reutlingen (DE); Kaleta, Christoph, 24248 Mönkeberg (DE); Renz, Alina, 72127 Kusterdingen (DE); Dräger, Andreas, 72138 Kirchentellinsfurt (DE); Hohner, Mirjam, 72074 Tübingen (DE); Best, Lena, 24105 Kiel (DE); Josephs-Spaulding, Jonathan, 24105 Kiel (DE)
(74) Representative: Witte, Weller & Partner Patentanwälte mbB

(57) **Abstract**

The present invention relates to a new broad-spectrum antiviral compound, a pharmaceutical composition comprising said broad-spectrum antiviral compound, and to a method for the prophylactic and/or therapeutic treatment of an infection of a living being by viruses.

## Description

The present invention relates to a new broad-spectrum antiviral compound, a pharmaceutical composition comprising said broad-spectrum antiviral compound, and to a method for the prophylactic and/or therapeutic treatment of an infection of a living being by viruses.

### FIELD OF THE INVENTION

The present invention relates to the field of drug development, more particular the field of antiviral agents.

### BACKGROUND OF THE INVENTION

Viruses are one of the major causes of morbidity and mortality in the world. International travel and migrations, globalization of commerce, technology and industry, agriculture development or climate changes are favoring the emergence and reemergence of viruses that could easily spread and potentially become a pandemic.

History shows that virus pandemics repeatedly plagued humankind. In the last 100 years alone, there have been four major influenza pandemics and multiple epidemics, including the Spanish flu in 1918 with estimated 17 to 50 million deaths worldwide and the swine flu pandemic in 2009 with a case fatality rate of 0.016, both caused by the H1N1 influenza A virus, and the Asian and Hong Kong flu in 1957/58 and 1968/69 with one to four million deaths worldwide.

In December 2019, the announced outbreak of a novel coronavirus (CoV) from a seafood market in Wuhan, China, became public. This novel virus had alarming similarities to the Severe Acute Respiratory Syndrome virus (SARS-CoV), responsible for a global outbreak in 2002 and 2003. In 2012, another coronavirus spread in the Middle East, causing the Middle East Respiratory Syndrome (MERS), leading to 2,458 reported cases and a high mortality rate of 35%.

This historical review indicates that global virus pandemics and epidemics are recurrent, and more are likely to follow in the future. Moreover, the current situation illustrates that pandemics also significantly impact long-term socioeconomic well-being due to the widespread and long-lasting consequences of efforts to contain them. Therefore, along with vaccines the rapid development of effective treatment strategies is vital to reducing mortality and mitigation measures' long-term socioeconomic consequences.

Most approved antiviral treatments are typically virus specific. The traditional strategy in drug development is based in the development of molecules that specifically target a viral protein (direct acting antivirals; DAA). This "one virus one drug" strategy has been quite successful for certain viruses like HIV or hepatitis C virus (HCV).

However, e.g., the DAAs currently available against influenza are characterized by poor efficacy. Influenza viruses constantly change and antiviral resistance emerges.

Very few drugs or DDAs are known to effectively inhibit SARS-CoV-2. Paxlovid inhibits viral replication and is currently authorized for treatment of COVID-19 in the European Union. One further drug, Lagevrio which inhibits viral replication through mutagenesis is currently under evaluation though recent clinical trials only note a moderate protection from severe disease.

The one virus one drug strategy is not the most cost efficient approach. Furthermore, the use of DAAs can easily lead to the appearance of drug resistant viruses due to the elevated mutation rate of viruses.

Due to all these reasons, there is an urgent need for new and efficient antivirals. Treatment approaches that can inhibit viral replication of not only single but a broad array of viruses as first-line therapeutic approaches for novel emergent pathogens are a highly sought-after goal for preparing for future pandemics.

It is, therefore, a problem underlying the invention to provide a new anti-viral compound by means of which the drawbacks of current antivirals can be avoided or at least reduced. In particular, such an antiviral compound is to be provided which can be used not only to inhibit one particular virus, i.e. a virus of a certain species, but different viruses or viruses belonging to different species or even higher taxonomic groups.

### SUMMARY OF THE INVENTION

The problem underlying the invention is solved by the provision of phenformin and salts thereof, solvates thereof and solvates of the salts thereof, for use in the prophylaxis and/or treatment of an infection of a living being by a first virus and/or by at least a second virus, wherein the first and the at least second viruses belong to a different virus species.

The problem underlying the invention is also solved by the provision of phenformin and salts thereof, solvates thereof and solvates of the salts thereof, for use in the prophylaxis and/or treatment of an infection of a living being by at least one unknown virus.

According to the invention "phenformin" is a drug belonging to the biguanide class, having the chemical formula C₁₀H₁₅N₅, a molar mass of 205.265 g·mol⁻¹, the ATC code A10BA01 (WHO), and CAS number 114-86-3. It was marketed as an antidiabetic drug by Ciba-Geigy, but was withdrawn from most markets in the late 1970s due to an alleged high risk of lactic acidosis, which has been reported as fatal in 50% of cases.

The phenformin may exist in stereoisomeric forms (enantiomers, diastereomers). The invention therefore also encompasses the enantiomers or diastereomers and respective mixtures thereof. The stereoisomerically uniform constituents can be isolated in a known manner from such mixtures of enantiomers and/or diastereomers. If the phenformin may occur in tautomeric forms, the present invention encompasses all tautomeric forms.

Salts preferred for the purposes of the present invention are physiologically or pharmaceutically acceptable salts of the phenformin. Also encompassed, however, are salts which are themselves not suitable for pharmaceutical applications but can be used for example for the isolation or purification of the phenformin.

Examples of pharmaceutically acceptable salts of the phenformin include salts of inorganic bases like ammonium salts, alkali metal salts, in particular sodium or potassium salts, alkaline earth metal salts, in particular magnesium or calcium salts; salts of organic bases, in particular salts derived from cyclohexylamine, benzylamine, octylamine, ethanolamine, diethanolamine, diethylamine, triethylamine, ethylenediamine, procaine, morpholine, pyrroline, piperidine, N-ethylpiperidine, N-methylmorpholine, piperazine as the organic base; or salts with basic amino acids, in particular lysine, arginine, ornithine and histidine.

Examples of pharmaceutically acceptable salts of the phenformin also include salts of inorganic acids like hydrochlorides, hydrobromides, sulfates, phosphates or phosphonates; salts of organic acids, in particular acetates, formates, propionates, lactates, citrates, fumarates, maleates, benzoates, tartrates, malates, methanesulfonates, ethanesulfonates, toluenesulfonates or benzenesulfonates; or salts with acidic amino acids, in particular aspartate or glutamate.

Solvates for the purposes of the invention refer to those forms of the phenformin, which in the solid or liquid state form a complex by coordination with solvent molecules. Hydrates are a specific form of solvates in which the coordination takes place with water.

The phenformin may also be complexed, e.g. with iron, calcium, etc., in which the compound may act as a ligand, so that a corresponding complex is also subject of the present invention.

According to the invention, "prophylaxis" and "treatment" of a virus infection refer to any measure to prevent or mitigate or ameliorate the infection and/or disease-causing conditions as a result of an infection that has occurred. In general, "virus infection" can be understood as acute or chronic persistent. Also included is, therefore, the prophylaxis and/or treatment of late effects of infection, such as "chronic infections", "post-acute sequelae associated with persistent viral infection", and, in the case of an SARS-CoV-2 infection, "long covid" or "post covid".

According to the invention, the prophylaxis or treatment of an infection "by a first virus and/or a second virus, which belong to a different virus species" means that said phenformin has broad-spectrum antiviral activity. As has been found by the inventors for the very first time said phenformin is effective against different viruses, i.e. not only against viruses of a certain virus species, but against viruses of different virus species or even higher taxonomic groups.

According to the invention, "unknown virus" refers to a virus for which the taxonomic affiliation is unclear or unknown.

A "living being" according to the invention includes any kind of living creature, including a vertebrate, a mammal, and a human being.

The finding by the inventors is surprising and could not have been expected when considering the prior art.

Lehrer S., Inhaled biguanides and mTOR inhibition for influenza and coronavirus (Review), World Acad Sci J. 2022;2(3): 1-11, and Grau-Expósito J. et al., Evaluation of SARS-CoV-2 entry, inflammation and new therapeutics in human lung tissue cells, PLoS Pathog 18(1):1-27, suggest the inhalative administration of phenformin for the treatment of a SARS-CoV-2. The authors attribute the therapeutic effect of phenformin in SARS-CoV-2 infection to its ability to inhibit the cellular protein mTOR ('mammalian target of rapamycin') via the so-called PI3K/AKT/mTOR pathway. The latter is described as playing a role in the infection of the related MERS-CoV; see Kindrachuk J. et al., Antiviral potential of ERK/MAPK and PI3K/AKT/mTOR signaling modulation for Middle East respiratory syndrome coronavirus infection as identified by temporal kinome analysis, Antimicrob Agents Chemother 2015;59: 1088-1099.

The present inventors were not able to verify the interaction of phenformin and mTOR as disclosed by Lehrer and Grau-Expósito et al. The inventors used an approach that significantly differs from the one described in the art. They developed a computational pipeline that uses single-cell RNA sequencing data mapped on a model of human metabolism to reconstruct the metabolic state of virally infected cells. The inventors used this pipeline to identify cellular factors which are essential for numerous viruses for a successful infection and/or proliferation in their hosts. By doing so the present inventors identified the NADH:ubiquinone oxidoreductase as an essential factor for virus replication. The NADH:ubiquinone oxidoreductase, also known as respiratory complex I, is the first large protein complex of the respiratory chains of many organisms from bacteria to humans. Phenformin has the capability to efficiently and selectively inhibit the NADH:ubiquinone oxidoreductase. The inventors recognized that, on the one hand, NADH:ubiquinone oxidoreductase is universally distributed across host species. They have also recognized that, on the other hand, a variety of viruses of diverse taxonomic affiliations must utilize the respiratory complex for successful infection. Only on the basis of these findings the inventors were able to conclude that phenformin is not an antiviral substance with a narrow action profile, as assumed by Lehrer and Grau-Expósito et al., but a substance with suitability as a broad-spectrum antiviral agent. Therefore, the inventors identified a new clinical situation which can be addressed by phenformin, such as a virus infection where the taxonomic affiliation or nature of the infectious virus is unknown. In addition, the inventors realized that phenformin can be used for the treatment or prophylaxis of infections by several, non-related viruses, for the treatment of which several different antiviral substances had to be used in the state of the art so far. The invention therefore helps to save the amount and number of antiviral substances. This not only reduces side effects, but also the costs of antiviral therapy.

The inventors also found out that phenformin can be administered in a certain therapeutic range where its antiviral activity is ensured but there is no cytotoxicity or the latter is significantly reduced, respectively. This was even more surprising. Because of the history of this active agent and the withdrawal from the markets due to an alleged high risk of lactic acidosis such an advantageous safety profile could not be expected.

In an embodiment of the invention the first and the at least second viruses belong to a different virus genus, preferably a different virus family, further preferably a different virus order, further preferably a different virus class, and still further preferably a different virus phylum.

This measure has the advantage that spectrum of activity of phenformin is significantly increased and does not only extend to viruses of different species but also to viruses of more distant relation. E.g., the inventors were able to demonstrate that phenformin has anti-viral activity against a virus belonging to the phylum of *Kitrinoviricota,* such as the dengue virus, but also against a virus belonging to the phylum of *Pisuviricota,* such as SARS-CoV-2.

Against this background, in an embodiment of the invention, the first virus is severe acute respiratory syndrome coronavirus 2 (SARS-CoV-2) or dengue virus (DENV). In another embodiment of the invention, wherein the at least second virus is dengue virus (DENV) or severe acute respiratory syndrome coronavirus 2 (SARS-CoV-2).

By this measure the compound according to the invention is adapted for the treatment of non-related viruses. SARS-CoV-2 is a strain of coronavirus that causes COVID-19 (coronavirus disease 2019), the respiratory illness responsible for the ongoing COVID-19 pandemic. It is a positive-sense single-stranded RNA virus that is contagious in humans. Dengue virus (DENV) is the cause of dengue fever. It is a mosquito-borne, single positive-stranded RNA virus.

In another embodiment of the invention said phenformin is configured for an oral administration to said living being.

This measure has the advantage that the active ingredient is provided in a dosage form that allows easy ingestion by the living being to be treated, e.g. a human patient. In this regard, the suitability of phenformin for oral administration was surprising based on the information in Lehrer (loc. cit.) and Grau-Expósito et al. (loc. cit.). There it is alleged that phenformin causes considerable side effects when administered systemically and, therefore, it would only be suitable for inhalation. However, by determining the therapeutic window, the inventors were able to establish that, at a suitable dosage, high antiviral activity is also achieved with oral administration, while at the same time minimizing cytotoxicity.

Another subject-matter of the invention relates to a pharmaceutical composition for use as a broad-spectrum antiviral comprising phenformin and the salts thereof, the solvates thereof and the solvates of the salts thereof according to the invention, and a pharmaceutically acceptable carrier.

"Pharmaceutically acceptably carriers" are well known to the skilled person. They allow a proper formulation of the compound according to the invention and serve to improve the selectivity, effectiveness, and/or safety of drug administration. Pharmaceutically acceptable carriers include, without being limited thereto, solvents, fillers, binders, lubricants, stabilizers, surfactants, suspensions, thickeners, emulsifiers, preserving agents, liposomes, micelles, microspheres, nanoparticles, etc. suitable for the particular form of dosage. Except for cases, when the medium of conventional carriers is incompatible with the active ingredient, for example, upon occurrence of any undesirable biological effects or other adverse interactions with any other ingredient(s) of the pharmaceutical composition, the use of such compositions falls within the scope of this invention. Materials that can serve as pharmaceutically acceptable carriers include, but are not limited to, monosaccharides and oligosaccharides, as well as derivatives thereof; malt, gelatin; talc; excipients such as: cocoa butter and suppository waxes; oils, such as peanut oil, cottonseed oil, safflower oil, sesame oil, olive oil, corn oil and soybean oil; glycols such as propylene glycol; esters, such as ethyl oleate and ethyl laurate; agar; buffering agents, such as magnesium hydroxide and aluminium hydroxide; alginic acid; pyrogen-free water; isotonic saline, Ringer's solution; ethyl alcohol and phosphate buffer solutions. In addition, the composition may contain other non-toxic compatible lubricants, for example sodium lauryl sulfate and magnesium stearate, as well as coloring agents, parting liquids, film formers, sweeteners, flavoring additives and flavorants, preserving agents and antioxidants.

The phrase "pharmaceutically acceptable" refers to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio. Suitable pharmaceutical carriers or excipients as well as pharmaceutical necessities for use in pharmaceutical formulations are described in Remington - The Science and Practice of Pharmacy, 23rd edition, 2020, a well-known reference text in this field, and in the USP/NF (United States Pharmacopeia and the National Formulary). Other sources are also available to one of ordinary skill in the art.

The features, characteristics, embodiments and advantages explained for the phenformin according to the invention apply *mutatis mutandis* to the pharmaceutical composition of the invention.

In a preferred embodiment of the invention said pharmaceutical composition is configured for an oral administration to a living being.

This measure has the advantage that the pharmaceutical composition is provided in a dosage form that allows easy ingestion by the living being to be treated, e.g. a human patient. In this regard, the suitability of phenformin for oral administration was surprising based on the information in Lehrer (loc. cit.) and Grau-Expósito et al. (loc. cit.). There it is claimed that phenformin causes considerable side effects when administered systemically and is only suitable for inhalation. However, by determining the therapeutic window, the inventors were able to establish that, at a suitable dosage, high antiviral activity is also achieved with oral administration, while at the same time minimizing cytotoxicity.

In still another embodiment said phenformin is present per dosage unit of the pharmaceutical composition at a concentration that, upon administration into a living being, results in a concentration in the virus-infected cells which is approx. ≤ 2000 µM, preferably about ≤ 1000 µM,further preferably ≤ 500 µM,further preferably ≤ 100 µM,further preferably ≤ 50 µM,further preferably ≤ 25 µM,further preferably ≤ 10 µM,further preferably ≤ 5 µM,further preferably ≤ 4 µM,further preferably ≤ 3 µM,further preferably ≤ 2 µM,and further preferably ≤ 1 µM.

This measure takes into account the findings of the inventors that setting the right therapeutic window by using the specified concentrations ensures on the one hand that the antiviral activity of phenformin is effective and on the other hand any cytotoxic effects are minimized.

In another embodiment of the invention the pharmaceutical composition comprises a further active agent, preferably a further anti-viral active agent.

While the phenformin can be used as the only active agent, i.e. administered in a monotherapy, this measure has the advantage that the inclusion of an additional anti-viral agent may not only lead to an additive effect but to a synergistic effects resulting in a potentiation of the effect of phenformin.

Another subject-matter of the invention relates to a method for the prophylactic and/or therapeutic treatment of an infection of a living being by a first virus and/or by at least a second virus, wherein the first and the at least second viruses belong to a different virus species, comprising the administration of the phenformin or the salts thereof, the solvates thereof and the solvates of the salts thereof according to the invention, or the pharmaceutical composition according to the invention, to said living being.

Yet a further subject-matter of the invention relates to a method for the prophylactic and/or therapeutic treatment of an infection of a living being by at least one unknown virus, comprising the administration of the phenformin or the salts thereof, the solvates thereof and the solvates of the salts thereof according to the invention, or the pharmaceutical composition according to the invention, to said living being.

The features, characteristics, embodiments and advantages of the compounds according to the invention and of the pharmaceutical composition according to the invention apply *mutatis mutandis* to the methods of the invention.

In the methods according to the invention, in an embodiment the administration is an oral administration, as discussed further above for the compounds according to the invention.

It is to be understood that the before-mentioned features and those to be mentioned in the following cannot only be used in the combination indicated in the respective case, but also in other combinations or in an isolated manner without departing from the scope of the invention.

The invention is now further explained by means of embodiments and examples resulting in additional features, characteristics and advantages of the invention. The embodiments are of pure illustrative nature and do not limit the scope or range of the invention. The features mentioned in the specific embodiments are general features of the invention which are not only applicable in the specific embodiment but also in an isolated manner and in the context of any embodiment of the invention.

Reference is made to figures in which the following is shown.

### BRIEF DESCRIPTION OF THE FIGURES

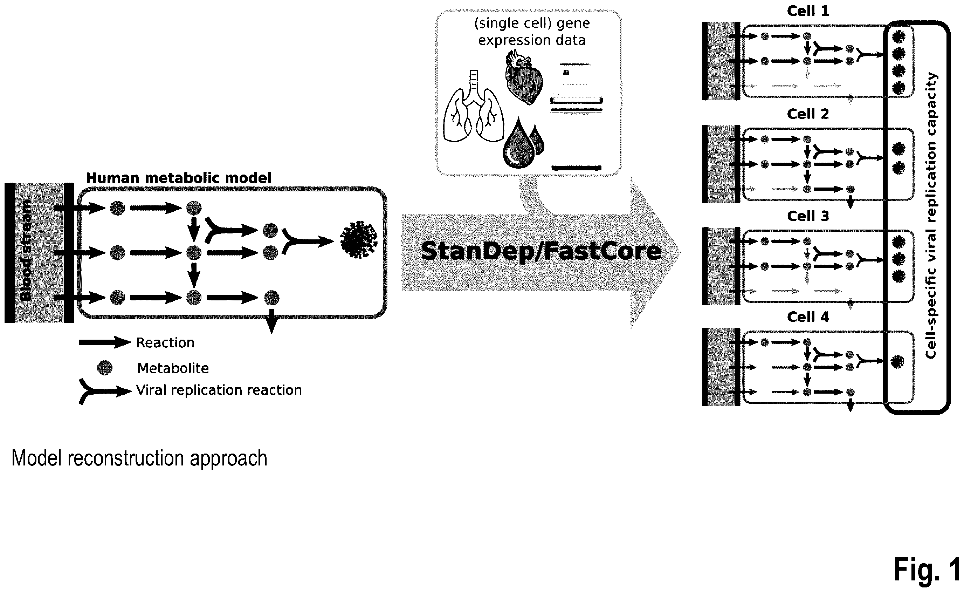

- Fig. 1: Illustration of the model used by the inventors to identify cellular viral targets. A computer model of human metabolism was expanded with metabolic reactions required for viral replication. Using transcriptomic data (single-cell or bulk sequencing) from virally infected cells/tissues, the metabolic state of said cells can be inferred. Subsequently, these viral-infection specific models can be used to determine enzyme knockouts that inhibit viral replication but do not disrupt normal cellular metabolism.

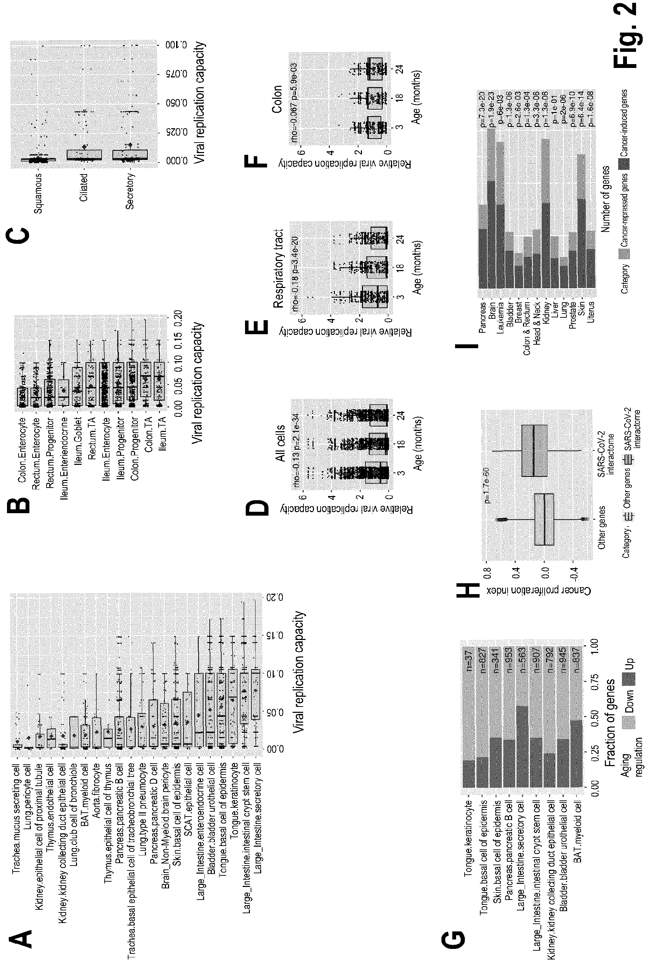

- Fig. 2: Viral replication capacity across cell types and age groups. Red circles indicate means of the distribution. A Viral replication capacity in mice across SARS-CoV-2 permissive cell types. B Viral replication capacity in SARS-CoV-2 permissive cell types of the human intestine. C Viral replication capacity in SARS-CoV-2 permissive cell types of the human lung. D-F Age-dependence of viral replication capacity across SARS-CoV-2 permissive cell types in mice (normalized across cell types, D - all cell types, E - cells of the respiratory tract, F - colonic cells). G Ageregulation of interaction partners of the SARS-CoV-2 proteome. Numbers indicate the total number of SARS-CoV-2 interacting proteins with a significant ageregulation for each cell type. H Cancer proliferation indices for SARS-CoV-2 interacting proteins. A positive value indicates genes whose expression is positively correlated with cancer growth rates and a negative value a negative correlation. P-value from a Wilcoxon rank-sum test of the cancer proliferation indices of SARS-CoV-2 interacting proteins against all other proteins. I Regulation of SARS-CoV-2 interacting proteins in cancer. Significantly up- and down-regulated genes in different types of cancer compared to matched normal controls from. P-values indicate the enrichment of SARS-CoV-2 interacting proteins among up-regulated genes versus down-regulated genes in the respective cancer type using Fisher's exact test.

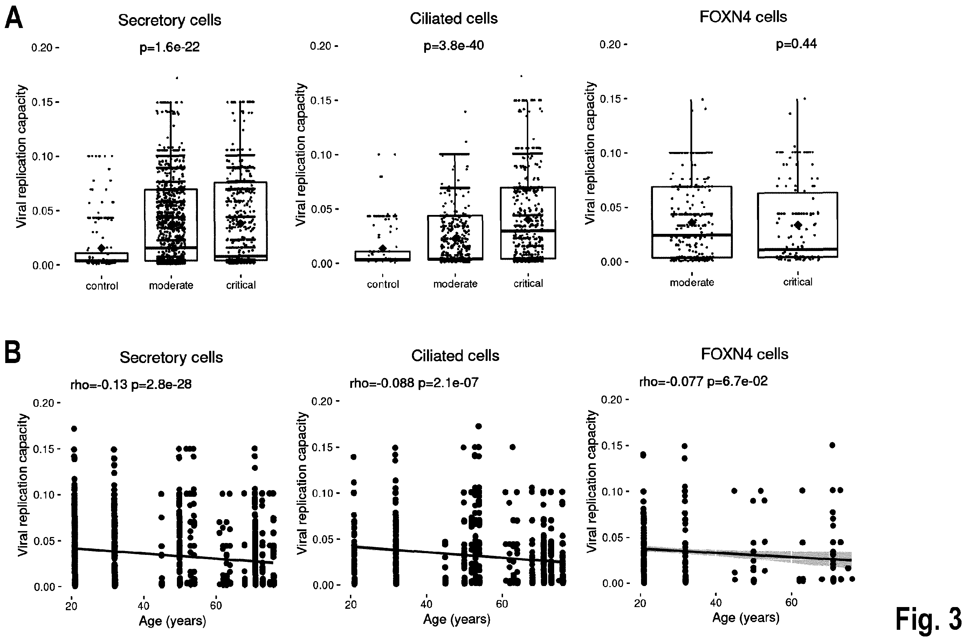

- Fig. 3:: Impact of disease state and age on viral replication capacity during infection. A Differences in viral replication capacity according to disease severity in SARS-CoV-2 permissive cell types of the lung. Please note that FOXN4-positive cells were only detected in infected individuals. B Impact of age group on viral replication capacity in SARS-CoV-2 infected patients.

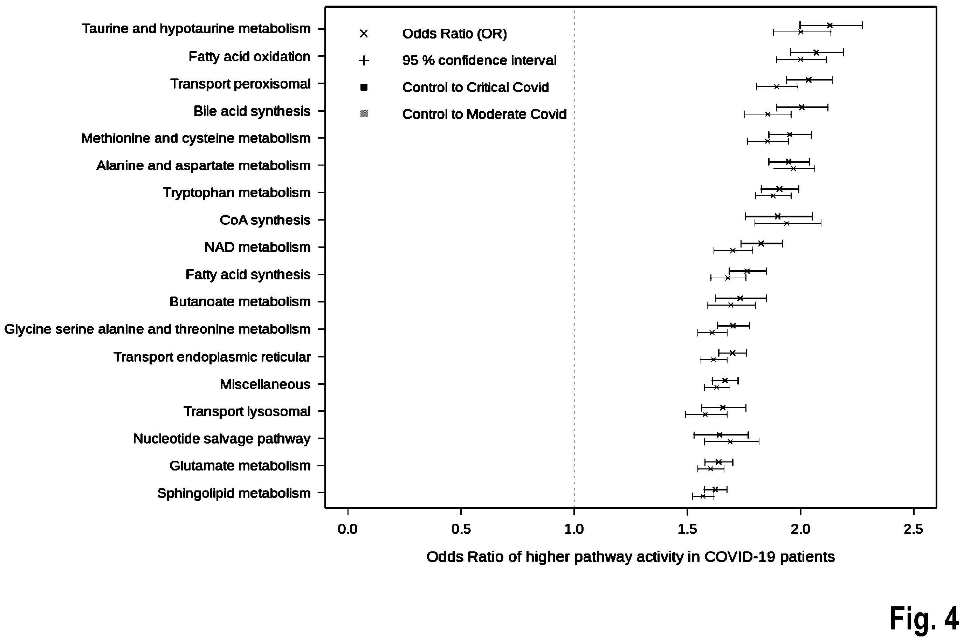

- Fig. 4:: Cellular metabolism is deregulated by viral infection. BALF2 lung lavage single-cell models were analyzed. Control to SARS-CoV-2 both severities (moderate and critical) controlled for cell type. Only cells where no viral RNA was detected were included (="uninfected cells"). All pathways appear to be more active in infected cells. Cells from patients with severe disease states show an even stronger pathway activation than cells from patients with moderate infection.

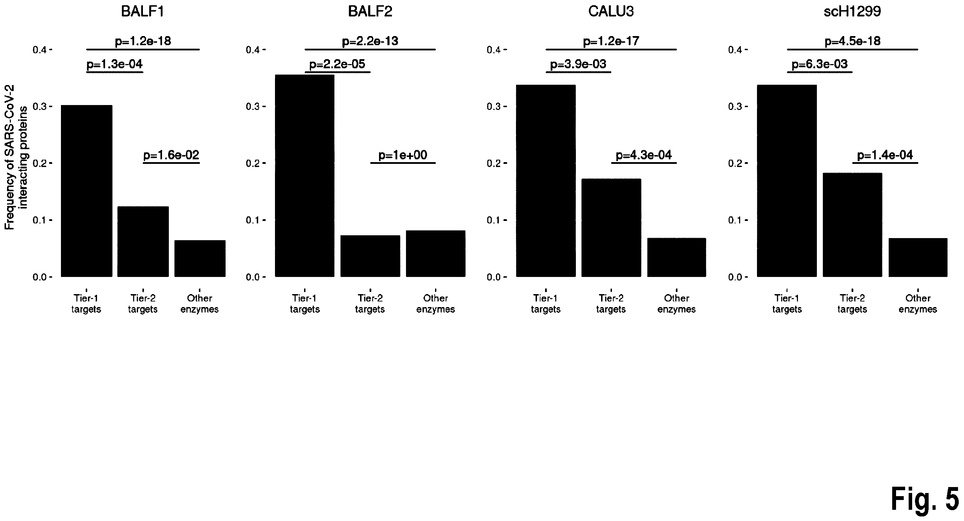

- Fig. 5:: Enrichment of SARS-CoV-2 interaction partners among enzymes relevant for viral replication in SARS-CoV-2-infected cells. For each data set, the fraction of genes that are experimentally-determined interaction partners of the SARS-CoV-2 proteome among computationally determined enzymes limiting viral replication versus all other metabolic genes is shown. Other enzymes correspond to those metabolic genes not part of the tier-1 or tier-2 targets. The significance of the enrichment has been determined using Fisher's exact test. P-values were corrected for multiple testing using false discovery rate control.

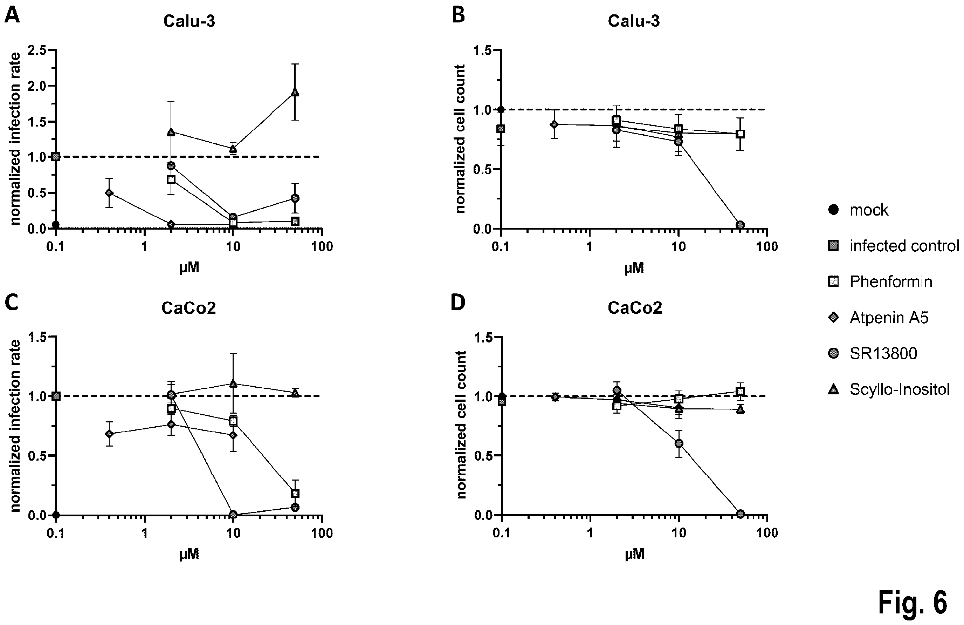

- Fig. 6:: Phenformin and Atpenin A5 show antiviral activity. Calu-3 (A), (B) and CaCo2 (C), (D) cells were pre-treated for 24h with Phenformin, Atpenin A5, SR13800 and Scyllo-Inositol in indicated concentrations and infected with icSARS-CoV-2-mNG. 48 hours post infection cells were fixed, stained with Hoechst and measured with Cytation3. (A), (C) Graphs show infection rate (mNG+/Hoechst+ cells) normalized to untreated infected cells. (B), (D) cell count (Hoechst+ cells) normalized to mock. n=3; error bars indicate SEM.

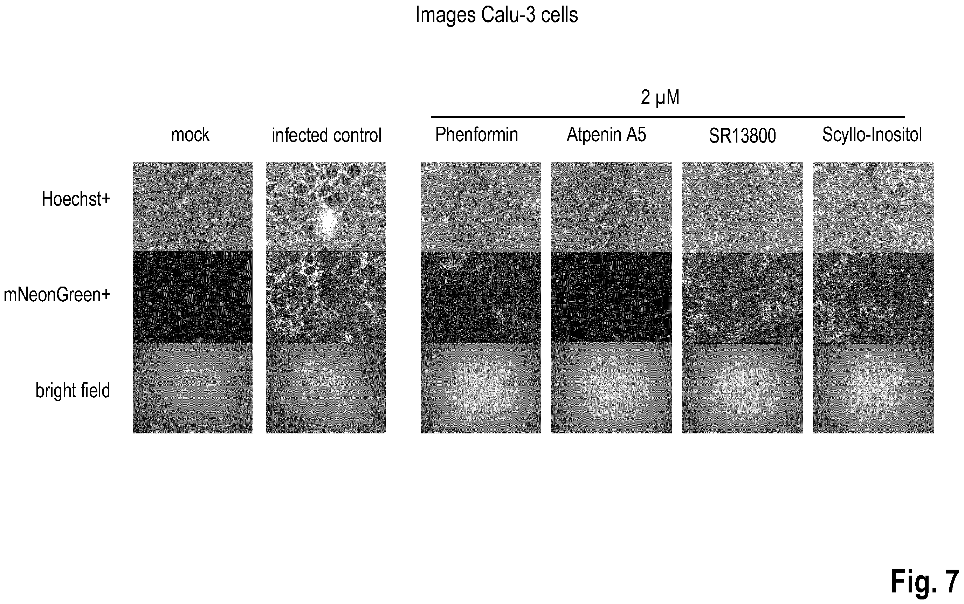

- Fig. 7:: Compound test in Calu-3 cells. Pre-treatment of Calu-3 cells with Phenformin, Atpenin A5, SR13800 and Scyllo-Inositol in 2 µM concentration for 24h, then infection with icSARS-CoV-2-mNG. 48 hours post infection cells were fixed, stained with Hoechst and measured with Cytation3. Representative fluorescence microscopy images taken at 4-fold magnification to detect cell nuclei count (Hoechst+), infected cells count (mNeonGreen+) and bright field images.

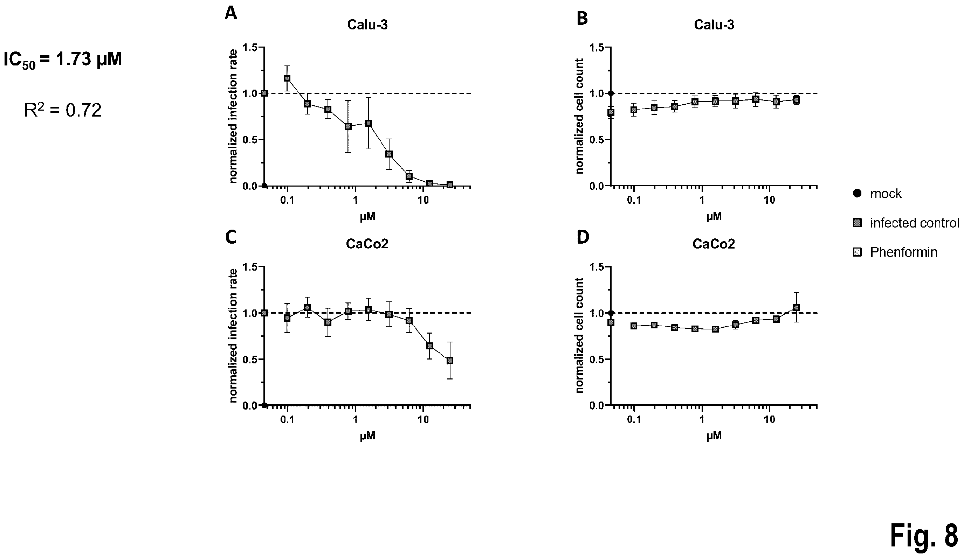

- Fig. 8:: IC₅₀ calculation in Calu-3 and CaCo2 cells. Calu-3 (A), (B) and CaCo2 (C), (D) cells were pre-treated for 24h with Phenformin in indicated concentrations and infected with icSARS-CoV-2-mNG. 48 hours post infection cells were fixed, stained with Hoechst and measured with Cytation3. (A), (C) Graphs show infection rate (mNG+/Hoechst+ cells) normalized to untreated infected cells. (B), (D) cell count (Hoechst+ cells) normalized to mock. n=3; error bars indicate SEM.

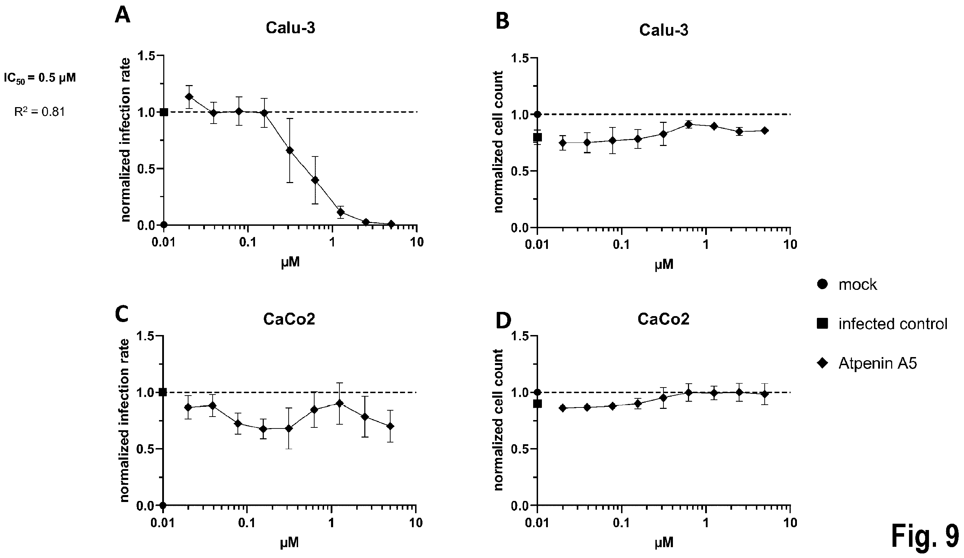

- Fig. 9:: IC₅₀ calculation in Calu-3 and CaCo2 cells. Calu-3 (A), (B) and CaCo2 (C), (D) cells were pre-treated for 24h with Atpenin A5 in indicated concentrations and infected with icSARS-CoV-2-mNG. 48 hours post infection cells were fixed, stained with Hoechst and measured with Cytation3. (A), (C) Graphs show infection rate (mNG+/Hoechst+ cells) normalized to untreated infected cells. (B), (D) cell count (Hoechst+ cells) normalized to mock. n=3; error bars indicate SEM.

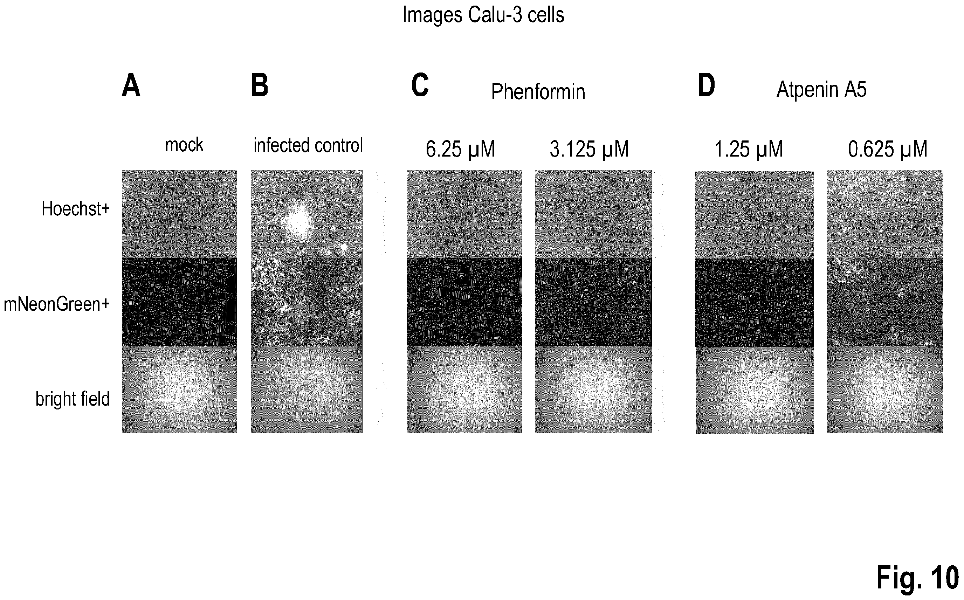

- Fig. 10:: Figure 5: IC₅₀ detection in Calu-3 cells. Pre-treatment of Calu-3 cells with Phenformin and Atpenin A5 for 24h, then infection of icSARS-CoV-2-mNG. 48 hours post infection cells were fixed, stained with Hoechst and measured with Cytation3. Representative fluorescence microscopy images taken at 4-fold magnification to detect cell nuclei count (Hoechst+), infected cells count (mNG+) and bright field images. (A) untreated and uninfected cells (mock) are shown. (B) untreated, infected cells are shown. (C) Phenformin pre-treated and infected cells. (D) Atpenin A5 pre-treated and infected cells.

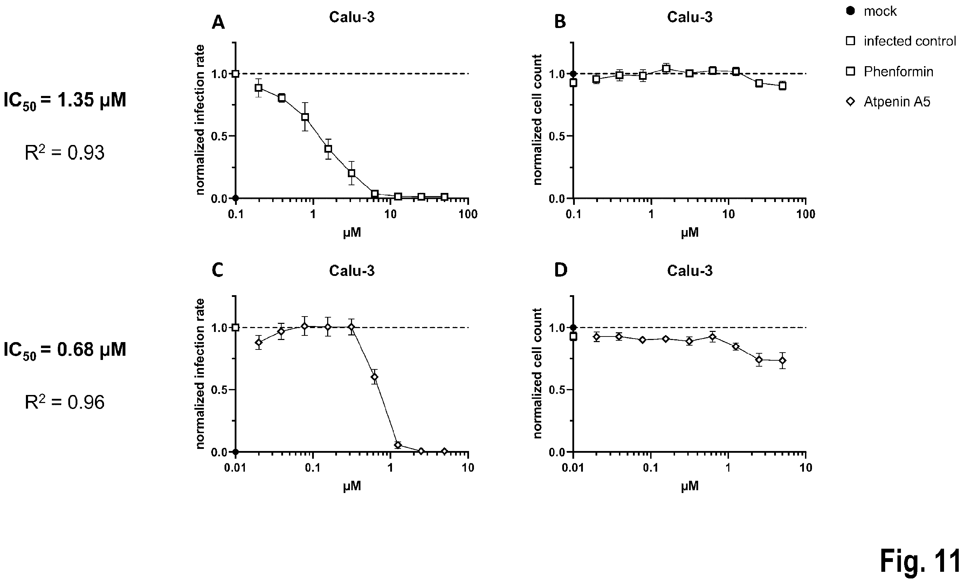

- Fig. 11:: IC₅₀ calculation in Calu-3 cells. Calu-3 cells were pre-treated for 24h with Phenformin (A), (B) and Atpenin A5 (C), (D) in indicated concentrations and infected with a SARS-CoV-2 clinical omicron variant. 48 hours post infection cells were fixed with 80% Acetone and IF staining was performed. Plates were measured by Cytation3. (A), (C) Graphs show infection rate (AlexaFluor594+ / DAPI cells) normalized to untreated infected cells. (B), (D) cell count (DAPI+ cells) normalized to mock. n=3; error bars indicate SEM.

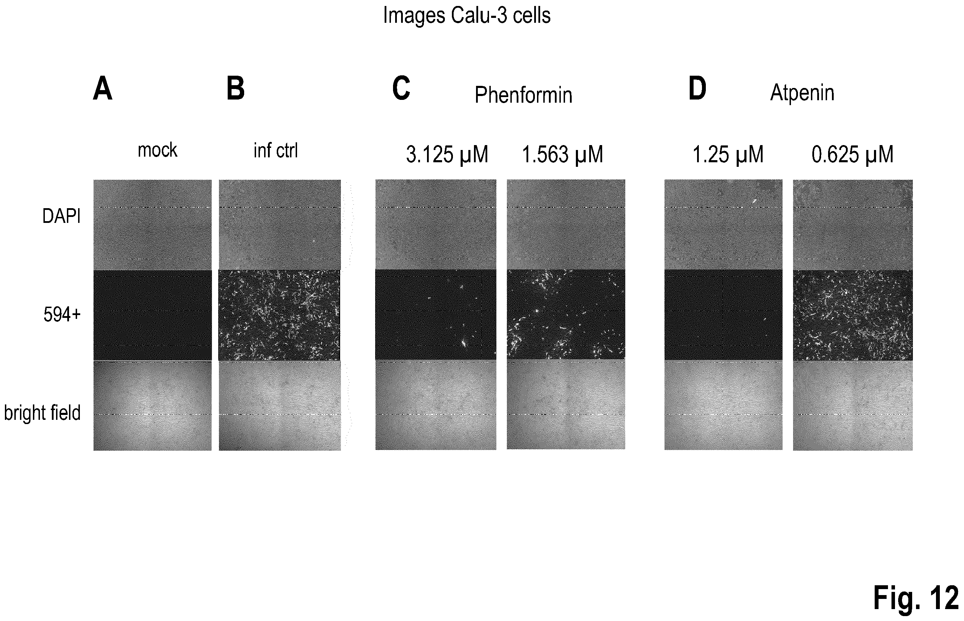

- Fig. 12:: IC₅₀ detection in Calu-3 cells. Pre-treatment of Calu-3 cells with Phenformin and Atpenin A5 for 24h, then infection with a SARS-CoV-2 clinical omicron isolate. 48 hours post infection cells were fixed with 80% Acetone and Immunofluorescence staining was performed. Cells were then measured with Cytation3. Representative fluorescence microscopy images taken at 4-fold magnification to detect cell nuclei count (DAPI+), infected cells count (AlexaFluor^{™} 594+) and bright field images. (A) untreated and uninfected cells (mock) are shown. (B) untreated, infected cells are shown. (C) Phenformin pre-treated and infected cells. (D) Atpenin A5 pre-treated and infected cells.

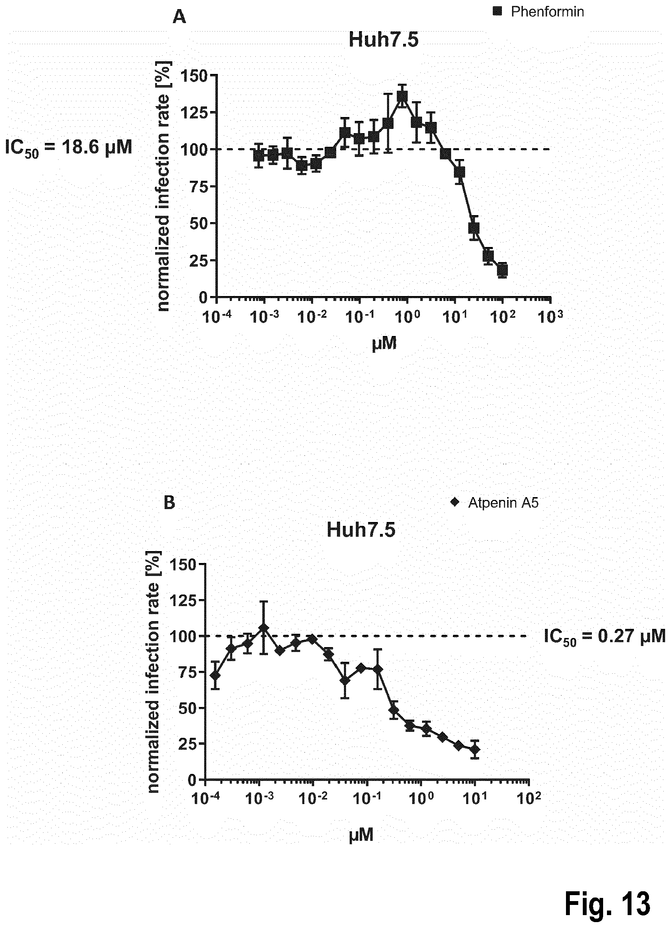

- Fig. 13:: IC₅₀ calculation in Huh7.5 cells. Huh7.5 cells were pre-treated for 1h with Phenformin (A) and Atpenin A5 (B) in indicated concentrations and infected with Dengue Virus. After 24h cells were washed and incubated for 10 minutes with RLuc lysis buffer. Cell lysates were transferred to a white measuring plate and RLuc assay buffer was added. Plates were measured by Cytation3. Graphs show Rluc Signal in procent normalized to infected control. n=3; error bars indicate SEM.

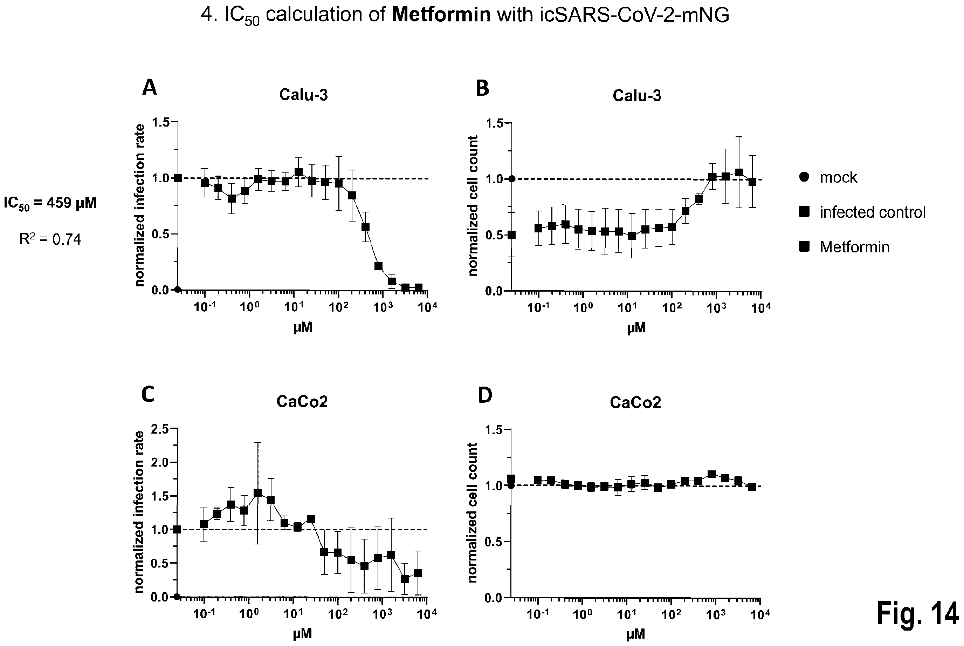

- Fig. 14:: IC₅₀ calculation in Calu-3 and CaCo2 cells. Calu-3 (A), (B) and CaCo2 (C), (D) cells were pre-treated for 24h with Metformin in indicated concentrations and infected with icSARS-CoV-2-mNG. 48 hours post infection cells were fixed, stained with Hoechst and measured with Cytation3. (A), (C) Graphs show infection rate (mNG+/Hoechst+ cells) normalized to untreated infected cells. (B), (D) cell count (Hoechst+ cells) normalized to mock. n=3, error bars indicate SEM.

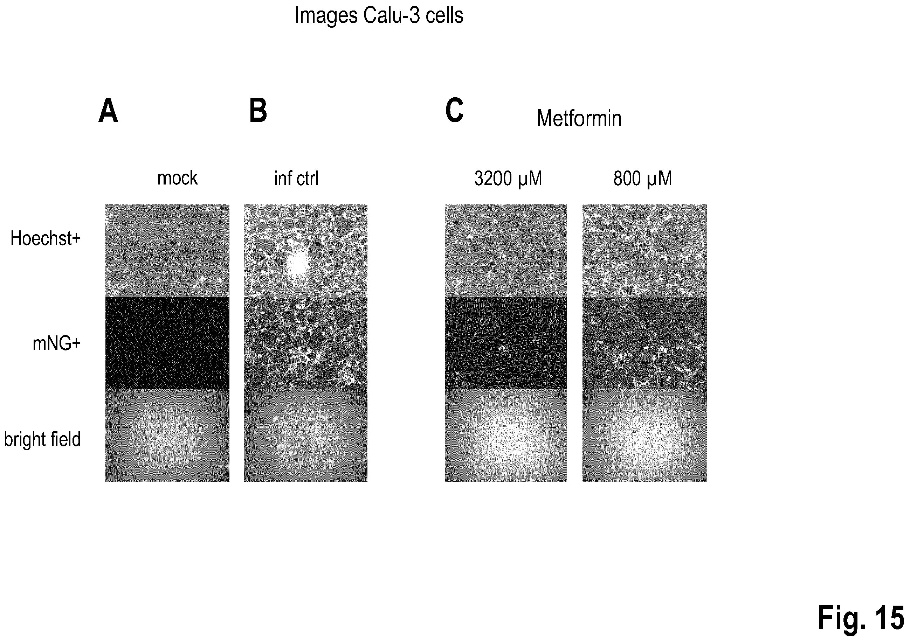

- Fig. 15:: IC₅₀ detection in Calu-3 cells. Pre-treatment of Calu-3 cells with Metformin for 24h, then infection of icSARS-CoV-2-mNG. 48 hours post infection cells were fixed, stained with Hoechst and measured with Cytation3. Representative fluorescence microscopy images taken at 4-fold magnification to detect cell nuclei count (Hoechst+), infected cells count (mNG+) and bright field images. (A) untreated and uninfected cells (mock) are shown. (B) untreated, infected cells are shown. (C) Metformin pre-treated and infected cells.

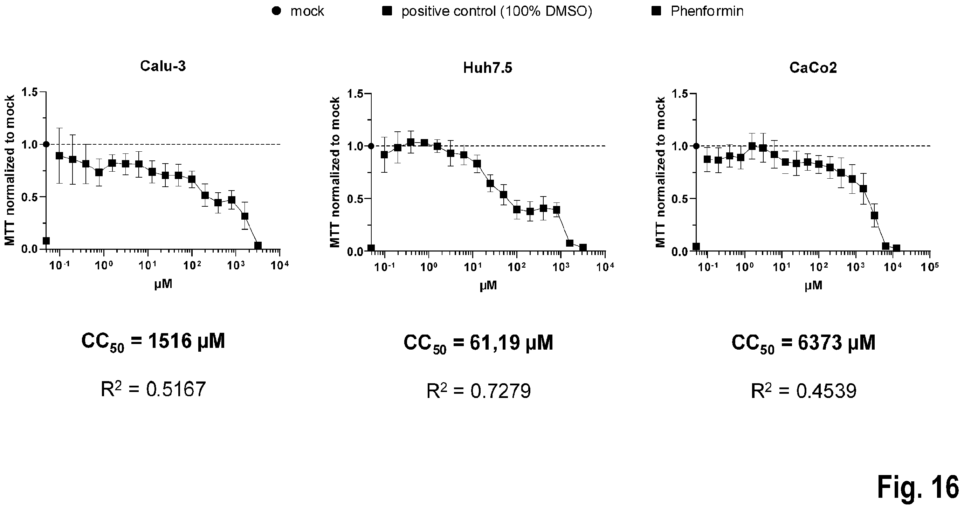

- Fig. 16:: Phenformin CC₅₀ calculation with MTT Assay. Calu-3, CaCo2 and Huh7.5 cells were treated with Phenformin in indicated concentrations. After 72h cells were incubated with phenolred-free DMEM Media and MTT solution for 3h, then supernatant was discarded, and cells were incubated with MTT buffer for 10 minutes. Absorption levels at 570 nm and 630 nm wavelength were measured by Berthold TriStar2 S. Graphs show normalized MTT signal to non-treated cells. n=3; error bars indicate SEM.

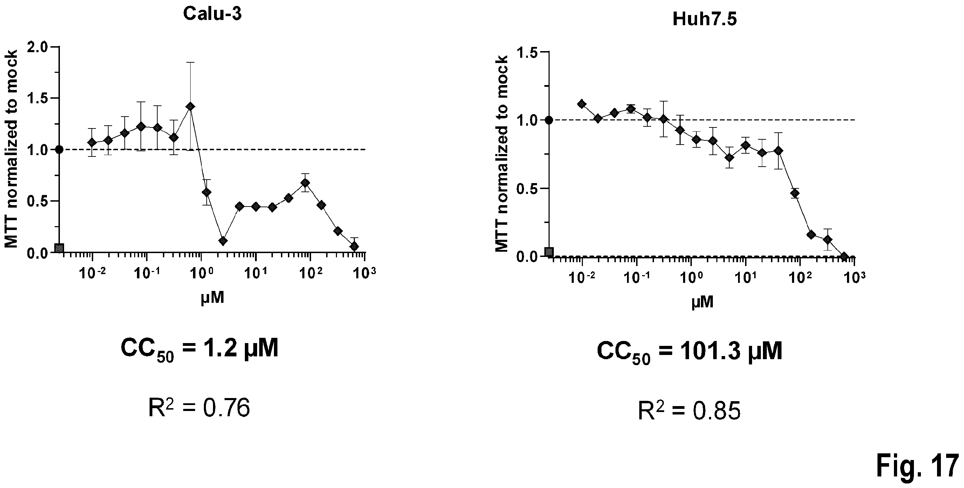

- Fig. 17:: Atpenin A5 CC₅₀ calculation with MTT Assay. Huh7.5 cells were treated with Atpenin A5 in indicated concentrations. After 72h cells were incubated with phenolred-free DMEM Media and MTT solution for 3h, then supernatant was discarded, and cells were incubated with MTT buffer for 10 minutes. Absorption levels at 570 nm and 630 nm wavelength were measured by Berthold TriStar2 S. Graphs show normalized MTT signal to non-treated cells. n=2 (Calu-3), n=3 (Huh7.5); error bars indicate SEM.

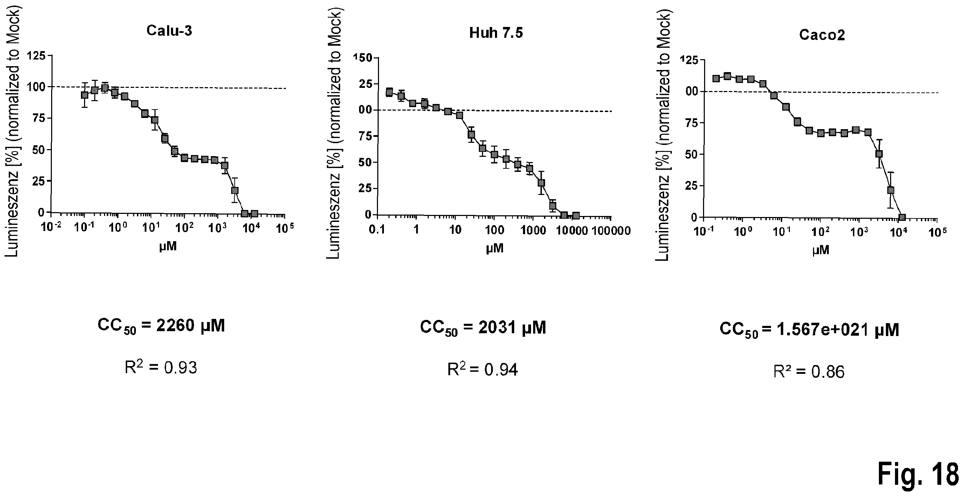

- Fig. 18:: Phenformin CC₅₀ calculation with CellTiterGlow^{®} Assay. Calu-3, CaCo2 and Huh7.5 cells were treated with Phenformin in indicated concentrations. After 72h cells were incubated with new DMEM- Media and CellTiterGlow^{®} reagent for 2 minutes (with shaking) and 10 minutes (without shaking). Luminescence Signal was measured by Berthold TriStar2 S. Graphs show normalized luminescence signal to non-treated cells. n=3; error bars indicate SEM.

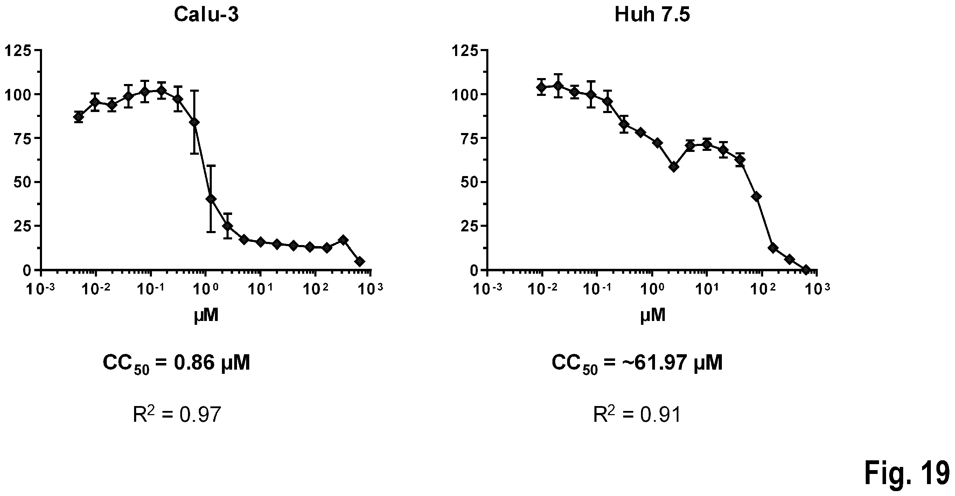

- Fig. 19:: Atpenin A5 CC₅₀ calculation with CellTiterGlow^{®} Assay. Calu-3 and Huh7.5 cells were treated with Atpenin A5 in indicated concentrations. After 72h cells were incubated with new DMEM-Media and CellTiterGlow^{®} reagent for 2 minutes (with shaking) and 10 minutes (without shaking). Luminescence Signal was measured by Berthold TriStar2 S. Graphs show normalized luminescence signal to non-treated cells. n=3; error bars indicate SEM.

### EXAMPLES

### 1. Introduction

The inventors developed a computational pipeline that allows them to reconstruct metabolic networks active in individual cells based on single-cell sequencing data from virally infected samples and cell cultures. In the context of SARS-CoV-2, the inventors used these networks to investigate cellular differences in the capacity to produce virions across a broad range of cell types. They found that primary sites of replication of SARS-CoV-2 in the human body are enriched for tissues with a particularly high viral replication capacity. Moreover, the inventors observed that viral infection leads to a considerable increase in viral replication capacity in non-infected bystander cells. Finally, the inventors used these models to predict intervention targets against SARS-CoV-2, which they experimentally confirmed in cell cultures.

An illustration of the model used by the inventors is depicted in Fig. 1.

### 2. Methods

### Single-cell sequencing datasets

Table 1 summarizes the single-cell sequencing datasets that were used.

**Table 1: Single-cell sequencing datasets**

| Dataset | Reference | Accession Number |
|---|---|---|
| BALF1 | Liao M. et al., Single-cell landscape of bronchoalveolar immune cells in patients with COVID-19. Nat Med. 2020;26: 842-844. | GSE 145926 |
| BALF2 | Chua RL. et al. COVID-19 severity correlates with airway epithelium-immune cell interactions identified by single-cell analysis. Nat Biotechnol. 2020;38: 970-979. | EGAS00001004481 |
| BALF3 | Bost P. et al. Deciphering the state of immune silence in fatal COVID-19 patients. Nat Commun. 2021;12: 1428. | GSE 157344 |
| CALU3 | Wyler E. et al. Transcriptomic profiling of SARS-CoV-2 infected human cell lines identifies HSP90 as target for COVID-19 therapy. iScience. 2021;24: 102151 . | GSE 148729 |
| scH1299 | Wyler E. et al. (loc. cit.) | GSE 148729 |
| FACS | Tabula Muris Consortium. A single-cell transcriptomic atlas characterizes ageing tissues in the mouse. Nature. 2020;583: 590-595. | GSE 109774 |
| Intestine | Wang Y. et al. Single-cell transcriptome analysis reveals differential nutrient absorption functions in human intestine. J Exp Med. 2020;217. doi: 10.1084/jem.20191130 | GSE 125970 |
| Airways | Deprez M. et al. A Single-Cell Atlas of the Human Healthy Airways. Am J Respir Crit Care Med. 2020;202: 1636-1645. | EGAD00001005714 |

### Blood serum diet

Blood Metabolites were accessed from the Human Metabolome Database (HMDB). Subsequently, an advanced query of blood metabolites from healthy adults was conducted in March 2019. During that time, a list of all human metabolites was obtained from the Virtual Metabolic Human (VMH) database. As VMH contains HMDB indices of the respective compounds, both were merged into a coherent database (Spring 2020). Drug-related or compounds without FooDB IDs were removed, and the nomenclature of included metabolites was modified to be compatible with an adapted version of the Recon 2.2 model. In correspondence to the mean values of the HMDB (cut-off values 10-6), all included values were transformed to mM. Water was set to 55,000 mM, and pH was assumed to be 7.4. The described calculations were conducted within the R packages sybil (version 2.1.5) and sybilSBML (version 3.0.5), in addition to their respective dependencies.

### scRNA core reaction pre-processing

Prior to reconstruction of single-cell metabolic models, gene expression scRNA datasets (Methods: scRNA Datasets) were accessed from NCBI's Gene Expression Omnibus (GEO) for pre-processing with Seurat and StanDep. Seurat objects of respective scRNA datasets were created, and quality control for the number of genes ( > 200 & < 6000) and mitochondrial (< 10) reads were standardized across all datasets. Normalized gene-level counts were extracted, converted into TPM-values, normalized according to human ENSEMBL gene lengths, and ENSEMBL gene names were mapped to Recon 2.2. StanDep was employed with the pre-processed expression data to identify core reaction genes across cell types, identifying both enzyme type and expression concerning Recon 2.2. Enzyme expression was then log10 transformed into a binary matrix with rows representing enzymes and columns as bins to identify minimum and maximum enzyme expression. A complete linkage metric with hierarchical clustering and Euclidean distance was used to cluster (number of clusters = 40) genes concerning their expression. Core reaction matrices were assembled, defined, and used as an input in FastCore to reconstruct single-cell, context-specific metabolic models.

### Viral replication model based on Recon 2.2

The cell-specific genome-scale metabolic models of cell-types relevant for SARS-CoV-2 infections are curated based on the human reconstruction Recon 2.2 using FastCore. With FastCore's fastcc algorithm and the simulated blood serum diet, a consistent model version of Recon 2.2 was created. A viral biomass objective function (VBOF) was generated for SARS-CoV-2, and the host biomass objective function (BOF) was defined as core reactions and, thus, included in all curated models. The FastCore algorithm was employed to create cell-type-specific models that were curated with the consistent model and a list of extended core reactions identified by StanDep with both BOF and VBOF.

### Prediction of antiviral targets

Subsequent analyses included optimizing the host's biomass maintenance and maximizing the viral biomass to assess the viral replication capacity of specific cell types. The inventors performed single gene deletions to predict antiviral targets and assess their effects on host maintenance and viral replication capacity. Gene knock-outs which were found to decrease viral replication capacity to a maximum of 50% of the initial value while maintaining the host's biomass minimally at 80% of its initial value were reported as potential antiviral targets.

Genes relevant for the viral replication capacity were identified by extracting genes whose knock-out resulted in a viral growth rate of less than 0.05% of its initial value while ignoring the knock-out effect in the host's maintenance functionality. The frequencies of the various potential antiviral targets within the host's metabolism were evaluated and ranked. Known virus-protein interactions and CRISPR-Cas studies were also used to rank the list of potential antiviral targets. The literature was searched for potential relevance of these genes in other virus diseases or cancer and known drugs or inhibitors. In addition to the literature research, different databases were browsed. To identify the gene's relevance in other virus diseases, the inventors searched in the database of protein, genetic and chemical interactions (BioGRID) and the molecular INTeraction database (MINT). For the gene's relevance in cancer, the MalaCards database and Cancer Genetics Web was browsed. To identify known drugs or inhibitors, the inventors searched in the Drug Gene Interaction database (DGIdb), the DrugBank database, and the Gene-Cards suite. Based on this ranked list, five potential antiviral targets were chosen for further evaluation in laboratory experiments.

### Screening of drug candidates

CaCo2 (10.000/well) and Calu3 (40.000/well) cells were seeded in 96-well plates in DMEM-Media containing 5% FCS. The cells were pre-treated for 24h with the drug candidates at the indicated concentrations. Then the pre-treated cells were infected with the respective virus strains at the indicated MOIs. 48 hours post infection the cells were fixed with 2% PFA and stained with Hoechst 1 µg/ml final concentration. Images of cell nuclei (390 nm; 460 nm), mNeonGreen (500 nm; 542 nm) and bright field were then taken with Cytation3 (Biotek, Winooski, VT, USA). Hoechst+ and mNeonGreen+ cells were counted by the Gen5 Software (Biotek, Winooski, VT, USA) and infection rates were calculated using the ratio of mNeonGreen+/Hoechst+ cells.

### IC₅₀ calculation of drug candidates

For IC₅₀ calculation of CaCo2 (10.000/well) and Calu-3 (40.000/well) cells were seeded in 96-well plates in DMEM-Media containing 5% FCS. The cells were pre-treated for 24h with the respective drug candidate in the indicated concentrations. Then the pre-treated cells were infected with the respective virus strains at the indicated multiplicity of infection (MOI). 48 hours post infection the cells were fixed with 2% PFA and stained with Hoechst 1 µg/ml final concentration. Images of cell nuclei (390 nm; 460 nm), mNeonGreen (500 nm; 542 nm) and bright field were then taken with Cytation3 (Biotek, Winooski, VT, USA). Hoechst+ and mNeonGreen+ cells were counted by the Gen5 Software (Biotek, Winooski, VT, USA) and infection rates were calculated using the ratio of mNeonGreen+/Hoechst+ cells). IC₅₀ was calculated as the half-maximal inhibitory dose via GraphPad Prism 9 using four-parameter nonlinear regression.

### CCso calculation of drug candidates

### MTT assay

Calu-3 (40.000/well), Huh7.5 (10.000/well) and CaCo2 (10.000/well) cells were seeded in 96-well plates in DMEM-Media. The cells were treated with drug candidates at the indicated concentrations. Negative control (non-treated), DMSO control and positive control (100% DMSO treated) were added, and plates were then stored at 37 °C, 5 % CO₂ for 72h. The old cell media was then discarded, and cells were incubated for 3 h at 37°C, 5% CO₂ with 90 µl phenolred-free DMEM-Media and 10 µl of a 5 mg/ml MTT solution per well (#ab146345 Abcam). Afterwards Media was discarded carefully, and Formazan crystals were dissolved on a shaker with 100 µl MTT buffer/well (0.04 N HCI in isopropanol) for 10 minutes. Absorption levels at 570 nm and 650 nm wavelength were measured by Berthold TriStar2 S Multimode Reader and values were normalized to non-treated cells. CC₅₀ was calculated as the half-maximal cytotoxic dose via GraphPad Prism 9 using four-parameter nonlinear regression.

### CellTiterGlow^{®} assay (CTG)

Calu-3 (40.000/well), Huh7.5 (10.000/well) and CaCo2 (10.000/well) cells were seeded in 96-well plates in DMEM-Media. The cells were treated with the drug compounds at the indicated concentrations, negative control (non-treated), DMSO control and positive control (100% DMSO treated) were added, and plates were then stored at 37 °C, 5 % CO₂ for 72h. The old cell media was then discarded, and cells were washed with PBS. 35 µl DMEM-Media and 35 µl CellTiterGlow^{®} reagent (#G7571, Promega) were added and incubated for 2 minutes on an orbital shaker protected from light. Afterwards plates were incubated for 10 minutes without shaking to stabilize the luminescent signal. 50 µl of the supernatant was transferred to a white 96 well measure plate and measured by Berthold TriStar2 S Multimode Reader. The background signal was determined by the luminescent signal of the positive control subtracted from the values used for analysis. The data was then normalized to the non-treated control. CC₅₀ was calculated as the half-maximal cytotoxic dose via GraphPad Prism 9 using four-parameter nonlinear regression.

### Monitoring cell viability via live cell imaging (IncuCyte^{®})

Calu-3 (40.000/well), Huh7.5 (10.000/well) and CaCo2 (10.000/well) cells were seeded in 96-well plates in DMEM-Media. The cells were treated with the drug compounds at the indicated concentrations, negative control (non-treated), DMSO control and positive control (50% DMSO treated) were added, and plates were then stored in the IncuCyte^{®} at 37 °C, 5 % CO₂ for 72h measuring every 3 hours cell confluency via phase channel with 10x objective. For Analysis IncuCyte^{®} S3 Software was used.

### 3. Results

### Cellular identity and age are important determinants of viral replication capacity

In an initial step, the inventors globally assessed cellular viral replication capacity across cell types that express the SARS-CoV-2 entry receptors ACE2 and TMPRSS2 (referred to as SARS-CoV-2 permissive cell types). To this end, the inventors reconstructed context-specific models based on the human metabolic network Recon 2.2 extended by a SARS-CoV-2-specific viral replication reaction conditioned with a medium derived from blood metabolite concentrations from the human metabolome database (see Methods). They used StanDep pre-processed scRNA-seq data for all cell types in the Tabula Muris Senis data set as input for the fastcore algorithm (see Methods) to build the context-specific models. This comprehensive mouse data set was used as a reference to avoid potential bias that is inherent in human data due to the often required post-mortem sample collection and to allow for an unbiased comparison of viral replication capacities across cell types. Following model reconstruction, the inventors predicted viral replication capacity for each cell using flux balance analysis.

Among SARS-CoV-2 permissive cell types, the inventors observed a broad range of viral replication capacities (Fig. 2A). The highest viral replication capacities were observed for the intestine and the oral cavity cells, which is in line with observations of viral replication in those tissues. The Tabula Muris dataset does not include the small intestine, which is assumed to be the primary site of viral replication in the gastrointestinal tract. Thus, the inventors built context-specific models of scRNA-seq data from humans covering the ileum, colon, and rectum from the gastrointestinal tract. In this data set, the inventors found the highest viral replication capacities in transit-amplifying (TA) cells across all tissues and enterocytes of the ileum (Fig. 2B). In line with the inventors' observation that intestinal cells are highly conducive to viral replication by SARS-CoV-2, it has been found that the small intestine of bats serves as a long-term reservoir for Coronaviridae. Similarly, viral proteins have been detected in small intestine biopsies from recovered patients several months after the clearance of viral infection in the respiratory tract. The inventors observed the highest viral replication capacity in alveolar type II pneumocytes in mouse lungs even though it was relatively small compared with other cell types (Fig. 2A). While coverage of SARS-CoV-2 permissive lung cell types was relatively low in the mouse data set, the inventors confirmed similar viral replication capacities in the upper respiratory tract of humans, including ciliated cells, which are considered the primary targets of SARS-CoV-2 infection (Fig. 2C).

Among other cell types demonstrating a high viral replication capacity, notable further examples are cells of the skin, pancreas, and brain pericytes. In agreement with the inventors' observations of a high viral replication capacity in the skin, skin lesions are reported in approximately 60% of COVID-19 patients, among which 75% of patients presented skin manifestation prior to clinical symptoms of COVID-19. Specifically, pediatric cases of skin manifestations are highly variable regarding frequency and severity, highlighting the likelihood of virus-associated active replication and immune response in the skin. Interestingly, the inventors observed a high viral replication capacity for brain pericytes. These cells corrugate the vasculature of the brain, muscles, and other organs and have previously been shown to be conducive to SARS-CoV-2 infection. While SARS-CoV-2 infection of patient brains has not been reported to date, the inventors' observations and the high frequency of neuronal symptoms in COVID-19 patients, specifically patients that demonstrate prolonged symptoms, indicate that brain pericytes represent a potential reservoir for viral infection.

Since age and age-related morbidities are important risk factors for severe outcomes in COVID-19, the inventors next determined how viral replication capacities change with age. In the mouse scRNA dataset, the inventors observed a significant reduction of viral replication capacity across age groups (Spearman rho=-0.13, p-value=2.1*10-34) and cell types (Fig. 2D). To exclude that a particular cell type drove the association, the inventors repeated the analysis 1000 times while excluding 50% of the cell types tested each time. In only 3 cases, we did not observe a significant (p<=0.05) negative association between viral replication capacity and age. By focusing on SARS-CoV-2 replication in different cell types, the inventors observed a strong negative association between viral replication rate and age in the respiratory tract (Fig. 2E) and to a lesser extent in the colon (Fig. 2F). To investigate whether down-regulation of the cellular viral replication capacity in the course of aging is exclusively a metabolic phenomenon, the inventors investigated the cell-specific aging-associated regulation of experimentally-determined interaction partners of the SARS-CoV-2 proteome in SARS-CoV-2 permissive cell types. Thus, the inventors observed a high percentage of these genes showed an aging-associated down-regulation for most cell types, indicating that the observation of a reduced cellular capacity to produce virions is not limited to the metabolic component of viral replication (Fig. 2G). Additionally, the inventors tested for an association between viral replication rate and sex but did not observe any differences in the human or mouse data.

Previously, it has been noted that viral infection entails similar metabolic changes to those observed in cancer. Thus, both metabolic changes and immune system responses to viral infection and cancer are similar. Previously, the inventors demonstrated a conserved age-associated regulation across species and tissues comprising a pronounced reversal of cancer-associated gene expression accompanied by an epidemiological decrease in cancer incidence in the very old. To explore whether an aging-associated down-regulation of cancer-associated pathways might explain the observed aging-associated reduction of viral replication capacity, the inventors determined the extent to which the SARS-CoV-2 interactome overlaps with genes deregulated in cancer. To this end, the inventors used the cancer proliferation index, which measures the correlation between the expression of each human gene with the growth rates of cancer cells from the NCI-60 cell lines. Considering experimentally determined interaction partners of the SARS-CoV-2 proteome, the inventors found that the SARS-CoV-2 interactome was highly enriched with genes whose expression was positively correlated with cancer cell growth (Fig. 2H). Thus, SARS-CoV-2 preferentially interacts with proteins that are also induced in cancer cells. While checking for the enrichment of the SARS-CoV-2 interactome with genes deregulated in cancer, the inventors observed a significant overlap between SARS-CoV-2 interacting proteins with cancer-induced genes across a large number of tissues (Fig. 2I). These results support the inventors' hypothesis that viral replication and cellular proliferation in cancer entail similar molecular requirements. Thus, the anti-cancer signature of the aging transcriptome may be the underlying reason for our observation of the reduction of viral replication capacity during aging.

### Viral infection and phenotypic parameters modulate viral replication capacity

Having investigated the viral replication capacity in healthy tissue, the inventors subsequently investigated how active SARS-CoV-2 infection impacted the predicted rate of viral replication in lung tissue. The inventors observed that the low level of viral replication in the upper respiratory tract cells in uninfected individuals was strongly increased in cells from SARS-CoV-2 infected individuals (Fig. 3A). During infection, both ciliated and secretory cells showed mean viral replication rates increased by a factor of 2 and 3, respectively. Both cell types are the primary site of cellular infection in the lungs. While viral replication rates may not be directly comparable between datasets, viral replication rates in cells from infected individuals reached similar levels to those in the gastrointestinal tract. Along those lines, it was also observed that ACE2-expression was also induced during viral infection. Since most cells in the data set did not show active viral replication, these results indicate that viral infection of a particular cell has pleiotropic effects on neighboring cells, making them much more conducive to viral infection. Some of these effects might be mediated by the previously observed virus-induced cellular senescence phenotype caused by SARS-CoV-2 infection. This cellular program entails a senescence- associated secretory phenotype that induces a pro-inflammatory microenvironment. However, to which extent these effects are mediated via direct effects (aberrant signalling by infected cells) or indirectly via interaction of SARS-CoV-2 with the immune system remains unclear.

After observing a generally reduced viral replication capacity with age, the inventors next investigated this association in scRNA-Seq data of upper airways from infected individuals. While controlling for sex, the inventors observed a strong negative association between age and viral replication rates in both ciliated and secretory cells (Fig. 3B). Interestingly, while the inventors predicted a reduced viral replication capacity with age at the baseline and for infected individuals, the amount of viral RNA detected in nasopharyngeal swabs has increased with age. However, there is no data yet whether this is due to a higher number of infected cells or an actual higher production of virions per cell. Concerning observations of an increased risk of severe disease associated with age, it is tempting to speculate that there is compensatory deregulation of cellular processes to compensate for reduced viral replication capacity with age. Specifically, SARS-CoV-2 infections may entail more potent deregulation of cellular processes in the elderly, leading to further tissue damage and stronger immune response. This assumption aligns with the inventors' observation that viral infection alone leads to a significant increase in viral replication capacity across cell types. Along those lines, mouse viral replication capacity was predicted to be the highest in intestinal cells, which show minor pathological changes in the presence of viral infection. Moreover, the inventors observed considerable changes in cellular metabolic activity in virally infected versus non-infected cells (Fig. 4).

### Enzymes essential for viral replication are enriched among the human SARS-CoV-2 interactome

Using the reconstructed metabolic models of individual SARS-CoV-2 infected cells, the inventors next investigated which enzymes are particularly relevant for efficient viral replication. To this end, the inventors knocked out each gene in the reconstructed models of infected cells and determined the resulting consequences for viral replication capacity using flux balance analysis. Additionally, the inventors determined whether cells were still able to form biomass following knockout. Subsequently, they separated enzymes into two classes: tier-1 targets and tier-2 targets. Tier-1 targets correspond to enzymes that reduce predicted viral replication by at least 50% while reducing cellular biomass production capacity by no more than 20%. In contrast, tier-2 targets completely abolish viral production but reduce cellular biomass production capacity by more than 20%. In principle, targets from the tier-1 group are desirable because while limiting viral replication, they are predicted not to limit normal cellular function by not impeding biomass production capacity.

To assess whether the derived targets are relevant for viral replication, the inventors determined interaction partners of the SARS-CoV-2 proteome for each set of genes as they would expect that viral proteins preferentially interact with host proteins relevant for viral replication. The inventors analyzed the four single-cell sequencing datasets (two patient data sets and two cell culture data sets). The inventors observed a highly significant enrichment of known interaction partners of the SARS-CoV-2 proteome among tier-1 and tier-2 targets compared to all other enzymes contained within the human metabolic model Recon 2.2 (Fig. 5). Interestingly, the inventors saw that tier-1 targets show a much stronger enrichment for known interaction partners than tier-2 targets. Tier-2 exclusive targets show only a slight enrichment compared to all other enzymes. From the perspective of viral replication, one would expect a preferential interaction with tier-2 targets since they are essential for viral replication, and lethality for the host cell should only be of minor relevance to the virus. However, it seems that SARS-CoV-2 preferentially interacts with proteins that, while being important for viral replication to a lesser extent, are essential for proper cellular functioning. Since the virus likely hijacks those enzymes to reprogram host cell metabolism for viral replication, this indicates that SARS-CoV-2 is reprogramming cellular metabolism to optimize viral replication while reducing the impact of cellular reprogramming on the normal functioning of the cell. One possible explanation for this somewhat contradictory behavior is that SARS-CoV-2 still requires a functioning cellular metabolism beyond an all-out production of virions while fostering viral replication. Alternatively, SARS-CoV-2 might try to minimize the cellular metabolic footprint of viral replication to reduce the likelihood of detection by the immune system, which can detect aberrant cellular metabolism as a sign of viral replication. Coronaviruses can usually maintain themselves in their bat hosts over long periods without apparent fitness effects for the host. Additionally, signs of viral replication have been detected in human patients even months after clearance of infection in the airways. These findings might indicate an immune-evasive strategy of SARS-CoV-2.

### Identification of drug targets

Following up on their identification of tier-1 and tier-2 targets for inhibiting viral replication, the inventors aimed to test the capability to identify drug candidates that could inhibit viral replication. To this end, the most frequently reported genes across the different single-cell sequencing datasets (see Table 1) were conjugated into a list containing 55 human genes. The genes were ranked based on known interactions with SARS-CoV-2, results from CRISPR-Cas experiments, their relevance in other viral diseases or cancer, and the availability of drugs or inhibitors to further narrow down this list. The top-ranking drug targets are listed in Table 2.

**Table 2: Potential antiviral targets. Based on single gene deletion analyses, potential antiviral targets were identified. The most frequent targets were ranked based on literature research. These five potential drug targets were further investigated in laboratory experiments.**

| **Target** | **Drug/Compound** |
|---|---|
| NADH:ubiquinone oxidoreductase | Metformin |
| | Phenformin |
| SLC16A1 | SR13800 |
| SLC1A5 | P-nitrophenyl analog 8 |
| SDHB | Atpenin |
| CDIPT | Scyllo-Inositol |

Twelve genes were associated with the NADH:ubiquinone oxidoreductase, coding for various subunits (A5, A6, A9, A10, A11, A13, B4, B9, B10, core S1, core S2, core S3). Each subunit interacts with one or two SARS-CoV-2 proteins. The two subunits A11 and B4 were also identified as relevant host factors for SARS-CoV-2 infections in a CRISPR-Cas knock-out screen. The subunit B10 (NDUFB10) plays a role in respiratory syncytial virus (RSV) infections, interacts with the human immunodeficiency virus 1 (HIV-1) [47], and the human gammaherpesvirus 8 (HHV-8) envelope glycoprotein. The core subunit S1 (NDUFS1) interacts with the influenza A virus and the human papillomavirus (HPV) serotype 18. It furthermore serves as a biomarker in renal cell carcinomas.

Core subunit S2 (NDUFS2) interacts with the hepatitis C virus (HCV) core protein and the Nipah virus' N-protein. The core subunit S3 (NDUFS3) plays a role in breast carcinomas, neuroblastomas, ovary adenomas, and adenocarcinomas. As SARS-CoV-2, SARS-CoV proteins also interact with subunit A10 (NDUFA10). This subunit is also downregulated by the human RSV. An overexpression of subunit A6 (NDUFA6) inhibits HIV-1, and a knock-out of subunit A9 (NDUFA9) enhances viral replication in several virus diseases. Subunit B9 interacts with HHV-8, and its downregulation promotes breast cancer proliferation. With these cross-references to other viral infections and cancer and its versatile interactions with SARS-CoV-2 proteins, the NADH:ubiquinone oxidoreductase seems a promising target for antiviral therapies. An already approved drug is metformin, which inhibits the NADH:ubiquinone oxidoreductase. Metformin is used to treat type II diabetes mellitus and belongs to the class of biguanides. The investigation of biguanides led to the development of the biguanide phenformin. Despite its withdrawal from the market based on the risk for lactic acidosis, research on this drug is still carried out, as its uptake rate into human cells is increased compared to metformin.

The solute carrier family 16 member 1 gene (SLC16A1), also known as monocarboxylate transporter (MCT), was identified as an interaction partner of HCV. It is reported to play a role in medulloblastomas, neuroblastomas, and malignant cutaneous melanomas. It is also a relevant host factor for SARS-CoV-2 infections in two CRISPR-Cas knock-out screens. Gamma-hydroxybutyric acid (GHB) and SR13800 were identified as potential inhibitors of SLC16A1, where GHB inhibits the MCT2 isoform and SR13800 the MCT1 isoform. The latter was chosen to be tested for its antiviral effect.

Solute carrier family 1 member 5 (SLC1A5) is a neutral amino acid transporter (ASCT2) and interacts with two SARS-CoV-2 proteins. The encoded protein also interacts with proteins from several HPV serotypes, a HIV-1 protein, the non-structural protein 2 from SARS-CoV, the HCV genotype 1b. The human endogenous virus type W (HERV-W) uses this transporter as a cell surface receptor. SLC1A5 does not only play a role in other viral infections, but it is also relevant in numerous cancer types. In colorectal cancer, e.g., SLC1A5 upregulation promotes cell growth and survival, while its inhibition sensitizes to cetuximab therapy. As this gene is highly relevant in several viral diseases and carcinosis and the P-nitrophenyl analog 8 is reported as an inhibitor, this potential antiviral target is highly interesting.

SARS-CoV-2 also interacts with the succinate dehydrogenase complex iron-sulfur subunit B (SDHB). Besides its interaction with SARS-CoV-2, SDHB also interacts with the Epstein-Barr virus (EBV), the Influenza A virus, HCV, and HHV-8. It is further investigated in correlation with adrenocortical cancers, head and neck cancers, pheochromocytomas, paragangliomas, gastrointestinal cancers, and kidney cancers. Atpenin is considered an SDHB inhibitor and was tested for its antiviral effect against SARS-CoV-2.

The last potential antiviral target is the CDP-diacylglycerol-inositol 3-phosphatidyltransferase (CDIPT). It is reported to interact with one SARS-CoV-2 protein and was reported in three CRISPR-Cas studies as a relevant host factor. CDIPT also interacts with proteins of numerous HPV serotypes and EBV. Due to its detection in multiple recent studies and its relevance in SARS-CoV-2, it is considered a potential antiviral target. Scyllo-inositol has potential efficacy in preventing Alzheimer's disease and is listed as an inhibitor of CDIPT.

These drugs or inhibitors for five targets were validated using viral infection assays.

### Initial screening of four drug candidates

CaCo2 and Calu3 cells were pre-treated with Phenformin, SR13800 and Scyllo-Inositol in concentrations of 50 µM,10 µM and 2 µM and Atpenin A5 in concentrations of 10 µM,2 µM and 0.4 µM.Then the pre-treated cells were infected with the SARS-CoV-2 strain icSARS-CoV-2-mNG at a multiplicity of infection (MOI) = 0.2 for CaCo2 cells and at a MOI = 0.5 for Calu-3 cells or mock-infected. The cells were then stained with Hoechst. Images of cell nuclei, mNeonGreen and bright field were then taken with Cytation3. Hoechst+ and mNeonGreen+ cells were counted and infection rates were calculated using the ratio of mNeonGreen+/Hoechst+ cells.

The experimental setup is shown in the following Table 3.

**Table 3: Experimental setup for initial screening of drug candidates**

| Calu3/C aCo2 | | 40 .000/10.000 ce ells/well date | | | | | | | |
|---|---|---|---|---|---|---|---|---|---|
| | | Inv ventors seed the the cells in 100µl, 1 day later add 1 100 µl of cpds for 24h, 4h, then add virus mNG-SARS ,-cov- | | | | | | | |
| | | 2 and and leave for 48 h 8 h | | | | | Conc centration = µM | | |
| PBS | PBS | | PBS | PBS | PBS | PBS | PBS | PBS | PBS |
| PBS | Phen. 50 µM | | Phen. 50 µM | Phen. 50 µM | SR 50 µM | SR 50 µM | SR 50 µM | mock | PBS |
| PBS | Phen. 10 µM | | Phen. 10 µM | Phen. 10 µM | SR 10 µM | SR 10 µM | SR 10 µM | mock | PBS |
| PBS | Phen. 2 µM | | Phen. 2 µM | Phen. 2 µM | SR 2 µM | SR 2 µM | SR 2 µM | mock | PBS |
| PBS | Scyllo 50 µM | | Scyllo 50 µM | Scyllo 50 µM | Atpenin 10 µM | Atpenin 10 µM | Atpenin 10 µM | inf control | PBS |
| PBS | Scyllo 10 µM | | Scyllo 10 µM | Scyllo 10 µM | Atpenin 2 µM | Atpenin 2 µM | Atpenin 2 µM | inf control | PBS |
| PBS | Scyllo 2 µM | | Scyllo 2 µM | Scyllo 2 µM | Atpenin 0,4 µM | Atpenin 0,4 µM | Atpenin 0,4 µM | inf control | PBS |
| PBS | PBS | | PBS | PBS | PBS | PBS | PBS | PBS | PBS |
| 48 phi, fix and stain cells (2 % PFA-Hoechst) 10 min and remove PFA | | | | | | | | | |

The result of this experiment is shown in Figs. 6 and 7

First, to evaluate the potential antiviral activity of the identified compounds Phenformin, AtpeninA5, SR13800 and Scyllo-Inositol the lung cell line Calu3 and the colon cell line Caco2 were infected with SARS-CoV-2 and infection rate measured by virus-encoded reporter gene expression (mNeonGreen) and cell viability assessed by Hoechst staining of the nucleus as described in the art. As shown in Fig. 6, Phenformin and ApteninA5 inhibited SARS-CoV-2 infection of the cell lines in a dose-dependent manner, whereas SR13800 was toxic and Scyllo-Inositol did not show any activity. Hence Phenformin and AtpeninA5 exert antiviral activity against SARS-CoV-2. Fig. 7 shows exemplary images of the aforementioned experiment at 48 hours post infection treated with 2 µM of the respective compounds.

### IC₅₀ calculation of Phenformin and Atpenin with icSARS-CoV-2-mNG

For IC₅₀ calculation of CaCo2 and Calu-3 cells were pre-treated with Phenformin in concentrations of 25 µM to 0.098 µM in two fold dilution and Atpenin A5 in concentrations of 5 µM to 0.02 µM in two fold dilution. Then the pre-treated cells were infected with the SARS-CoV-2 strain icSARS-CoV-2-mNG at a multiplicity of infection (MOI) = 0.2 for CaCo2 cells and at a MOI = 0.5 for Calu-3 cells or mock-infected. Then the cells were stained with Hoechst. Images of cell nuclei, mNeonGreen and bright field were then taken with Cytation3. Hoechst+ and mNeonGreen+ cells were counted and infection rates were calculated. IC₅₀ was calculated as the half-maximal inhibitory dose.

The experimental setup is shown in Table 4.

**Table 4: Experimental setup for IC₅₀ calculation of Phenformin and Atpenin with icSARS-CoV-2-mNG**

| Calu3/CaCo2 | 40.000/10.000 cells/well | | | | | | | | | | | |
|---|---|---|---|---|---|---|---|---|---|---|---|---|
| | Inventors seed the cells in 100µl, 1 day later add 100 µl of Atpenin for 24h, then add 10 µl virus mNG- | | | | | | | | | | | |
| | SARS-CoV-2 and leave for 48 h | | | | | | | | | Concentration = µM | | |
| | PBS | PBS | PBS | PBS | PBS | PBS | PBS | PBS | PBS | PBS | PBS | PBS |
| | PBS | 5 | 2,5 | 1,25 | 0,625 | 0,313 | 0,156 | 0,078 | 0,039 | 0,020 | mock | PBS |
| Atpenin | PBS | 5 | 2,5 | 1,25 | 0,625 | 0,313 | 0,156 | 0,078 | 0,039 | 0,020 | mock | PBS |
| | PBS | 5 | 2,5 | 1,25 | 0,625 | 0,313 | 0,156 | 0,078 | 0,039 | 0,020 | mock | PBS |
| | PBS | 25 | 12,5 | 6,25 | 3,125 | 1,563 | 0,781 | 0,391 | 0,195 | 0,098 | inf | PBS |
| | PBS | 25 | 12,5 | 6,25 | 3,125 | 1,563 | 0,781 | 0,391 | 0,195 | 0,098 | inf | PBS |
| Phenformin | PBS | 25 | 12,5 | 6,25 | 3,125 | 1,563 | 0,781 | 0,391 | 0,195 | 0,098 | inf | PBS |
| | PBS | PBS | PBS | PBS | PBS | PBS | PBS | PBS | PBS | PBS | PBS | PBS |
| 48 phi, fix and stain cells (2 % PFA-Hoechst) 10 min and remove PFA | | | | | | | | | | | | |

The result of this experiment is shown in Figs. 8 (Phenformin), 9 (Atpenin) and 10.

To evaluate the potency of Phenformin to suppress SARS-CoV-2 infection of Calu-3 and Caco-2 cells were treated with different concentrations and infection assessed 48 hours post infection. Then the IC₅₀ was calculated and toxicity of the compound evaluated by staining of the nuclei (Fig. 8). Phenformin had an IC₅₀ of 1.73 µM in Calu3 cells without any toxicity. Similarly, the IC₅₀ of AtpeninA5 was assessed (Fig.9) which was 0.5 µM in Calu3 cells. Exemplary primary microscopy images of the ICso-determination are shown in Fig.10.

### IC₅₀ calculation of Phenformin and Atpenin with SARS-CoV-2 omicron variant

For IC₅₀ calculation Calu-3 cells were pre-treated with Phenformin in concentrations of 50 µM to 0.195 µM and Atpenin A5 in concentrations of 5 µM to 0.02 µM in two-fold dilution. Then the pre-treated cells were infected with a SARS-CoV-2 clinical omicron isolate at a multiplicity of infection (MOI) = 1.1 or mock-infected. Then immunofluorescence staining was performed with rabbit anti-SARS-CoV-2 nucleocapsid antibody and incubated with goat anti-rabbit AlexaFluor^{™} 594 antibody. Images of cell nuclei, AlexaFluor^{™} 594+ cells and bright field were then taken with Cytation3. DAPI+ and AlexaFluor^{™} 594+ cells were counted and IC₅₀ was calculated as the half-maximal inhibitory dose.

The plate layout is shown in Table 5.

**Table 5: Plate layout for IC₅₀ calculation of Phenformin and Atpenin with SARS-CoV-2 omicron variant.**

| Calu3/CaCo2 | 40.000/we ell | | | | | | | | | | | |
|---|---|---|---|---|---|---|---|---|---|---|---|---|
| | Inventors seed the cells in 100µl, 1 day later r add 100 µl of cpd for 24h, then add 5 µl virus s SARS-C | | | | | | | | | | | |
| | omicron v variant and leave for 48 h | | | | | | | | | Conce ntration = µM | | |
| | PBS | PBS | PBS | PBS | PBS | PBS | PBS | PBS | PBS | PBS | PBS | PBS |
| | PBS | 5 | 2,5 | 1,25 | 0,625 | 0,313 | 0,156 | 0,078 | 0,039 | 0,020 | mock | PBS |
| Atpenin | PBS | 5 | 2,5 | 1,25 | 0,625 | 0,313 | 0,156 | 0,078 | 0,039 | 0,020 | mock | PBS |
| | PBS | 5 | 2,5 | 1,25 | 0,625 | 0,313 | 0,156 | 0,078 | 0,039 | 0,020 | mock | PBS |
| | PBS | 50 | 25 | 12,5 | 6,25 | 3,125 | 1,563 | 0,781 | 0,391 | 0,195 | inf | PBS |
| | PBS | 50 | 25 | 12,5 | 6,25 | 3,125 | 1,563 | 0,781 | 0,391 | 0,195 | inf | PBS |
| Phenformin | PBS | 50 | 25 | 12,5 | 6,25 | 3,125 | 1,563 | 0,781 | 0,391 | 0,195 | inf | PBS |
| | PBS | PBS | PBS | PBS | PBS | PBS | PBS | PBS | PBS | PBS | PBS | PBS |
| 48 phi, fix with 80 % Acetone and perform IF staining | | | | | | | | | | | | |

The result of this experiment is shown in Figs. 11 and 12.

The reporter virus used for assessment of antiviral activity thus far, is based on an early Wuhan strain of SARS-CoV-2. Therefore, the inventors aimed to evaluated in Phenformin and AtpeninA5 are also active against the currently circulating Omicron variant. Therefore, we repeated the experiment to determine the IC₅₀S of Phenformin and Atpenin in Calu3 cells exactly as before, but used a patient-derived Omicron isolate and stained for the viral nucleocapsid protein by immunofluorescence, to detect virally infected cells (Fig. 11 with representative images in Fig. 12). This revealed, that Phenformin (IC50 = 1.35 µM) and AtpeninA5 (IC50 = 0.68 µM) have similar antiviral activity also against the Omicron variant, indicating that it is active against all currently circulating variants of concern.

### IC₅₀ calculation of Phenformin, Atpenin and Metformin with Dengue virus

Phenformin, Atpenin and Metformin were prediluted in Huh7.5 complete media and added to Huh7.5 cells before adding the virus. Lysis buffer was added to the cells and the cell lysate was transferred to a white measuring plate. The plate was measured with the Cytation3 plate reader. The data was analyzed using the GraphPad Prism 9.

The results of this experiment are shown in Figs. 13 (Phenformin, Atpenin), 14 (Metformin) and 15.

Viruses in general, depend on basic cellular metabolic activity for robust intracellular genome replication and viral protein translation, assembly and egress. The inventors hence hypothesized that complex I inhibition by Phenformin might also inhibit distantly related viruses of other families. As an example, the inventors used Dengue virus (DENV) which is a highly pathogenic and emerging Flaviviruses causing several hundred million infections per year, with a large proportion of severe cases and at risk to die.

Here, the inventors used a DENV type 2 reporter virus expressing the Renilla Luciferase as infection reporter. Similarly, to the experiments with SARS-CoV-2, cells were pre-treated with the compounds, then infected and infection was assessed by measuring reporter gene activity 24 hours later (Fig. 13). This revealed that Phenformin had an IC₅₀ of 18.6 against DENV in Huh7.5 hepatoma cell lines while the complex 2 inhibitor Atpenin A5 had an IC₅₀ of 0.27 µM.Hence, Phenformin and Atpenin A5 are antiviral active against different non-related viruses.

Metformin is related to Phenformin and was reported to have antiviral activity in the mM range against DENV and ZIKA with a low therapeutic index. Furthermore, it was suggested to inhibit SARS-CoV-2, which has not been experimentally addressed. The inventors therefore evaluated the antiviral activity of Metformin against SARS-CoV-2 and found that it only exhibited antiviral activity at relatively high concentrations, with an IC₅₀ of 459 µM in Calu3 cells without any cytotoxicity (Fig. 14; see representative images Fig. 15). Therefore, while Metformin seems to harbor some antiviral activity, it is highly questionable if such high concentrations of the compound can be achieved in the respective target tissues.

### CC₅₀ calculation of Phenformin and Atpenin

### a) MTT assay

Calu-3, Huh7.5 and CaCo2 cells were treated with Phenformin in concentrations of 12800 µM to 0.097 µM and Atpenin A5 in concentrations of 640 µM to 0.0098 µM in two fold dilution. Absorption levels at 570 nm and 650 nm wavelength were measured and values were normalized to non-treated cells. CC₅₀ was calculated as the half-maximal cytotoxic dose via GraphPad Prism 9 using four-parameter nonlinear regression.

The result is shown in Fig. 16 (Phenformin) and 17 (Atpenin).

Phenformin acts on the complex 1 and Atpenin A5 on complex 2. Hence, even though the inventors' drug identification pipeline is designed to yield cellular targets, that can be inhibited to suppress virus replication without harming the host cells, they wanted to carefully evaluate the potential toxicity of their compounds. They furthermore aimed to establish the CC₅₀ of their compounds to calculate the therapeutic index (CC₅₀ / IC₅₀), which is an indicator of the selective inhibition of a target (i.e. a virus) by compound over its toxic effects.

In all the previous experiments, based on counting nuclei of living cells, the inventors already established that the compounds do not show any toxicity on virally infected cells in the concentrations used. They now expanded the tests and used MTT assay, which is a procedure used to calculate the redox activity at the mitochondrial membrane of cells and the commercial cell titer glow assay (Promega) to measure cellular ATP. Cell lines tested were the ones used for our antiviral assay, i.e. Calu-3, Caco-2 and Huh7.5 cells.

MTT revealed that all cell types tolerated very high doses of Phenformin with a CC₅₀ of 1516 µM in Calu-3 cells, 61 µM in Huh7.5 and 6373 in Caco-2 (Fig. 16), Atpenin A5, maybe expected due to its complex 2 inhibition, showed a CC₅₀ of 1.2 µM in Calu3 and 101.3 µM in Huh7.5 when measured via MTT (Fig. 17).

### b) CellTiterGlow^{®} assay (CTG)

Calu-3, Huh7.5 and CaCo2 (10.000/well) cells were treated with Phenformin in concentrations of 12800 µM to 0.097 µM and Atpenin A5 in concentrations of 640 µM to 0.0098 µM in two fold dilution. Luminescent signals were measured by Berthold TriStar2 S Multimode Reader. The data was then normalized to the non-treated control. CC₅₀ was calculated as the half-maximal cytotoxic dose via GraphPad Prism 9 using four-parameter nonlinear regression.

The result is shown in Fig. 18 (Phenformin) and 19 (Atpenin).

Consistent with the MTT data, Phenformin had a CC₅₀ higher than 2 µM for all cell lines with Cell Titer glow (Fig. 18) and Atpenin A5 and Phenformin also mirrored the MTT data with CC₅₀S of 0.86 µM in Calu-3 and 61.97 µM in Huh7.5 (Fig. 19). Hence while Atpenin A5 as expected shows activity related to complex 2 dependent processes, Phenformin seems highly tolerated in cells and does not induce any severe cytotoxicity.

### c) Monitoring cell viability via live cell imaging (IncuCyte^{®})

Calu-3, Huh7.5 and CaCo2 (10.000/well) cells were treated with Phenformin in concentrations of 12800 µM to 0.097 µM and Atpenin A5 in concentrations of 640 µM to 0.098µM in two fold dilution. Plates were then stored in the IncuCyte^{®} measuring every 3 hours cell confluency. For Analysis IncuCyte^{®} S3 Software was used.

Finally, both compounds were excessively tested by live-cell imaging and determination of growth curves firmly establishing a low level of growth inhibition by both compounds that is above 1000 µM for Phenformin and 60 µM for Atpenin A5 (data not shown and Tables 6 and 7).

### Summary of ICso, CCso and the therapeutic range of Phenformin and Atpenin

Values of IC₅₀, CC₅₀ and the therapeutic range of Phenformin and Atpenin found by the inventors are summarized in the following Tables 6 and 7.

**Table 6: IC₅₀, CC₅₀ and the therapeutic range of Phenformin**

| Phenformin | IC₅₀ | CC₅₀ | Therapeutic range: CC₅₀/IC₅₀ |
|---|---|---|---|
| ic-SARS-CoV-2-mNG (in Calu-3 cells) | 1.73 µM | MTT: 1516 µM | MTT: 876.3 |
| | | CTG: 2260 µM | CTG: 1306.4 |
| | | IncuCyte: 1500 µM | IncuCyte: 867.1 |
| SARS-CoV-2 omicron (in Calu-3 cells) | 1.35 µM | MTT: 1516 µM | MTT: 1123 |
| | | CTG: 2260 µM | CTG: 1674 |
| | | IncuCyte: 1500 µM | IncuCyte: 1111 |
| Dengue Virus (in Huh7.5 cells) | 18.6 µM | MTT: 61.19 µM | MTT: 3.3 |
| | | CTG: 2031 µM | CTG: 109.2 |
| | | IncuCyte: 1115 µM | IncuCyte: 59.9 |

**Table 7: IC₅₀, CC₅₀ and the therapeutic range of Atpenin**

| Atpenin | IC₅₀ | CC₅₀ | Therapeutic range: CC₅₀/IC₅₀ |
|---|---|---|---|
| ic-SARS-CoV-2-mNG (in Calu-3 cells) | 0.5 µM | MTT: 1.2 µM | MTT: 24 |
| | | CTG: 0.86 µM | CTG: 1.72 |
| | | IncuCyte: > 160 µM | IncuCyte: > 320 |
| SARS-CoV-2 omicron (in Calu-3 cells) | 0.68 µM | MTT: 1.2 µM | MTT: 1.8 |
| | | CTG: 0.86 µM | CTG: 1.3 |
| | | IncuCyte: > 80 µM | IncuCyte: > 117.6 |
| Dengue Virus (in Huh7.5 cells) | 0.27 µM | MTT: 101.3 µM | MTT: 375.2 |
| | | CTG: 61.97 µM | CTG: 229.5 |
| | | IncuCyte: 65 µM | IncuCyte: 240.7 |

The measured IC₅₀ and CC₅₀ values for Phenformin and Atpenin A5 throughout suggest that these compounds have a high on-target activity (which is the virus) but show low to no toxicity towards the infected cells (therapeutic range is higher than 100 throughout). From this, a high therapeutic index is calculated that allows a broad range of compound concentrations to be used prospectively in patients. Atpenin A5 and Phenformin only in Huh7.5 cells, shows test specific lower values, which are likely caused by their mode-of-action, which is targeting complex 1 and 2, but ultimately does not lead to cellular cytotoxicity but robust inhibition of virus replication.

## Claims

1. Phenformin and salts thereof, solvates thereof and solvates of the salts thereof, for use in the prophylaxis and/or treatment of an infection of a living being by a first virus and/or by at least a second virus, wherein the first and the at least second viruses belong to a different virus species.

2. The phenphormin and the salts thereof, the solvates thereof and the solvates of the salts thereof of claim 1, wherein the first and the at least second viruses belong to a different virus genus, preferably a different virus family, further preferably a different virus order, further preferably a different virus class, and still further preferably a different virus phylum.

3. The phenphormin and the salts thereof, the solvates thereof and the solvates of the salts thereof of claim 1 or 2, configured for an oral administration to said living being.

4. The phenformin and the salts thereof, the solvates thereof and the solvates of the salts thereof of any of the preceding claims, wherein the first virus is severe acute respiratory syndrome coronavirus 2 (SARS-CoV-2) or dengue virus (DENV).

5. The phenformin and the salts thereof, the solvates thereof and the solvates of the salts thereof of any of the preceding claims, wherein the at least second virus is dengue virus (DENV) or severe acute respiratory syndrome coronavirus 2 (SARS-CoV-2).

6. Phenformin and salts thereof, solvates thereof and the solvates of the salts thereof, for use in the prophylaxis and/or treatment of an infection of a living being by at least one unknown virus.

7. A pharmaceutical composition for use as a broad-spectrum antiviral comprising phenformin and the salts thereof, the solvates thereof and the solvates of the salts thereof according to any of claims 1-6 and a pharmaceutically acceptable carrier.

8. The pharmaceutical composition of claim 7, configured for an oral administration to a living being.

9. The pharmaceutical composition of claim 7 or 8, wherein said phenformin is present per dosage unit at a concentration that, upon administration into a living being, results in a concentration in the virus-infected cells which is approx. ≤ 2000 µM,preferably about ≤ 1000 µM,further preferably ≤ 500 µM,further preferably ≤ 100 µM,further preferably ≤ 50 µM,further preferably ≤ 25 µM,further preferably ≤ 10 µM,further preferably ≤ 5 µM,further preferably ≤ 4 µM,further preferably ≤ 3 µM,further preferably ≤ 2 µM,and further preferably ≤ 1 µM.

10. The pharmaceutical composition of any of the preceding claims, wherein it comprises a further active agent, preferably a further anti-viral active agent.

11. A method for the prophylactic and/or therapeutic treatment of an infection of a living being by a first virus and/or by at least a second virus, wherein the first and the at least second viruses belong to a different virus species, comprising the administration of the phenformin or the salts thereof, the solvates thereof and the solvates of the salts thereof of any of the claims 1-8, or the pharmaceutical composition of any of the claims 8-10 to said living being.

12. A method for the prophylactic and/or therapeutic treatment of an infection of a living being by at least one unknown virus, comprising the administration of the phenformin or the salts thereof, the solvates thereof and the solvates of the salts thereof of any of the claims 1-8, or the pharmaceutical composition of any of the claims 8-10 to said living being.
